(19)

(11) **EP 4 759 903 A1**

(12) **EUROPEAN PATENT APPLICATION**
published in accordance with Art. 153(4) EPC

(43) Date of publication:
**17.06.2026 Bulletin 2026/25**

(21) Application number: **24851937.3**

(22) Date of filing: **08.08.2024**

(51) International Patent Classification (IPC):
***C10G 1/10*** *(2006.01)* ***B01J 29/40*** *(2006.01)*
***B01J 37/04*** *(2006.01)* ***B01J 37/08*** *(2006.01)*
***C08J 11/16*** *(2006.01)*

(52) Cooperative Patent Classification (CPC):
**B01J 29/40; B01J 37/04; B01J 37/08; C08J 11/16; C10G 1/10;** Y02W 30/62

(86) International application number:
**PCT/JP2024/028573**

(87) International publication number:
**WO 2025/033520 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.08.2023 JP 2023131007**

(71) Applicant: **Resonac Corporation**
**Tokyo 105-7325 (JP)**

(72) Inventors:
- **UEDA, Yasuyuki**
  **Tokyo 105-7325 (JP)**
- **MINAMI, Takuya**
  **Tokyo 105-7325 (JP)**
- **TAKASHI, Hiroko**
  **Tokyo 105-7325 (JP)**
- **TEZUKA, Noriyasu**
  **Tokyo 105-7325 (JP)**
- **SATO, Takashi**
  **Tokyo 105-7325 (JP)**

(74) Representative: **Hoffmann Eitle**
**Patent- und Rechtsanwälte PartmbB**
**Arabellastraße 30**
**81925 München (DE)**

(54) **METHOD FOR PRODUCING OLEFIN-CONTAINING COMPOSITION AND CATALYST FOR PRODUCING OLEFIN-CONTAINING COMPOSITION**

(57) A method for producing an olefin-containing composition includes a step of decomposing plastic in the presence of a zeolite to produce an olefin-containing composition including an olefin with 2 to 5 carbon atoms, wherein the zeolite is an MFI zeolite including $Rb^+$.

EP 4 759 903 A1

## Description

### TECHNICAL FIELD

The present invention relates to methods for producing olefin-containing compositions and catalysts for producing olefin-containing compositions.

### BACKGROUND ART

Plastic waste has been processed through landfill, ocean dumping, incineration, or the like. However, it is getting more difficult to secure landfill sites, and ocean dumping causes environmental problems as plastic is not decomposed. Further, although heat from incineration of plastic can be utilized, incineration has a problem associated with emission of carbon dioxide gas, which leads to global warming.

Therefore, recycling of plastic waste, such as reuse, regeneration, and the like, has been desired in recent years from rising awareness in environmental issues. Research and development on plastic waste recycling have been actively carried out. In addition, most plastic materials are produced by fossil fuels, and establishment of a recycling method is also strongly desired in view of effective use of resources.

Among plastic materials, hydrocarbon-based plastic materials, such as polyethylene (PE), polypropylene (PP), polystyrene (PS), and the like are widely used for drink and food containers, packaging materials, molded article, films, and the like because of excellent properties, such as heat resistance, weather resistance, mechanical strength, transparency, chemical resistance, gas barrier properties, and the like. Therefore, the hydrocarbon-based plastic materials occupy the majority of the plastic materials constituting plastic material waste. As a recycling method for these plastic materials, there is chemical recycling that uses pyrolysis or gasification. In the pyrolysis or gasification, the plastic waste is turned into low molecular hydrocarbons by thermal decomposition, and many methods using a solid catalyst for improving reactivity or selectivity of resultant products have been studied.

For example, as a method for producing olefins that has an excellent yield of olefins with 2 to 3 carbon atoms and an excellent ratio between olefins and paraffins included in a catalytic decomposition product with 2 to 3 carbon atoms, a method for producing olefins, which includes a thermal decomposition step and a catalytic decomposition step is proposed. In the thermal decomposition step, a polyolefin-based plastic is heated to obtain a decomposition product. In the catalytic decomposition step, the decomposition product obtained in the thermal decomposition step is brought into contact with 0.10 percent by mass to 0.30 percent by mass of an MFI zeolite including sodium atoms to obtain a catalytic decomposition product (see Patent Document 1). Further, Ga-loaded zeolite (HZSM-5 and HZSM-11) catalysts are proposed as catalysts for achieving a high aromatic yield, and HZSM-5 and Na ion-exchanged NaHZSM-5 are proposed as catalysts having high selectivity of lower olefins (see Non-Patent Document 1).

### RELATED ART DOCUMENTS

#### PATENT DOCUMENTS

Patent Document 1: International Publication No. WO 2021/166854

#### NON-PATENT DOCUMENT

Non-Patent Document 1: Yoshio Uemichi and Yasuharu Kanda, "Hydrogen Transfer Degradation of Mixed Plastics Using Zeolite Catalysts", 2023, Vol. 65, No. 2, p. 114-119

### DISCLOSURE OF THE INVENTION

#### PROBLEMS TO BE SOLVED BY THE INVENTION

However, a yield of a useful component, such as propylene or the like, relative to a by-product, such as a paraffin component or the like, is not sufficiently satisfactory even when the catalysts disclosed in the related art documents are used.

The present disclosure aims to provide a method for producing an olefin-containing composition, which yields a small amount of paraffins as a by-product, and has an excellent yield of olefins with 2 to 5 carbon atoms.

## MEANS FOR SOLVING THE PROBLEMS

According to one aspect of the present disclosure, a method for producing an olefin-containing composition includes a step of decomposing plastic in the presence of a zeolite to produce an olefin-containing composition that includes an olefin with 2 to 5 carbon atoms, in which the zeolite is an MFI zeolite including $Rb^+$.

The means for solving the above problems are as follows.

<1> A method for producing an olefin-containing composition, the method including:
a step of decomposing plastic in the presence of a zeolite to produce an olefin-containing composition including an olefin with 2 to 5 carbon atoms,
wherein the zeolite is an MFI zeolite including $Rb^+$.
<2> The method for producing the olefin-containing composition according to <1>, wherein the plastic is polyolefin.
<3> The method for producing the olefin-containing composition according to <1>,
wherein the plastic includes at least one selected from the group consisting of polyethylene, polypropylene, and polystyrene.
<4> The method for producing the olefin-containing composition according to any one of <1> to <3>,
wherein the decomposing of the plastic is performed at a temperature of 300°C or higher and 1,100°C or lower.
<5> The method for producing the olefin-containing composition according to any one of <1> to <4>,
wherein a molar ratio [$SiO_2/Al_2O_3$] of $SiO_2$ to $Al_2O_3$ of the MFI zeolite including $Rb^+$ is 10 or greater and 10,000 or less.
<6> The method for producing the olefin-containing composition according to any one of <1> to <5>,
wherein the MFI zeolite including $Rb^+$ has a BET specific surface area of 100 $m^2/g$ or greater and 1,000 $m^2/g$ or less.
<7> The method for producing the olefin-containing composition according to any one of <1> to <6>, further including:

> a mixing step of obtaining a liquid mixture in which an MFI zeolite and a liquid including a rubidium compound are mixed; and
> a calcination step of calcining the liquid mixture in the air to obtain the MFI zeolite including $Rb^+$.

<8> The method for producing the olefin-containing composition according to <7>,
wherein the mixing step satisfies $1 \leq yz/x \leq 10{,}000$, where x (g) is a mass of the MFI zeolite, y is a molar ratio [$SiO_2/Al_2O_3$] of $SiO_2$ to $Al_2O_3$ of the MFI zeolite, and z (mmol) is a number of moles of the rubidium compound.
<9> The method for producing the olefin-containing composition according to <7> or <8>,
wherein the MFI zeolite is an MFI zeolite including $H^+$.
<10> A catalyst for producing an olefin-containing composition, the catalyst including:
an MFI zeolite including $Rb^+$,
wherein the catalyst is used for producing an olefin-containing composition that includes an olefin with 2 to 5 carbon atoms.
<11> A method of using a catalyst, the method including:
using the catalyst that includes an MFI zeolite including $Rb^+$ in production of an olefin-containing composition that includes an olefin with 2 to 5 carbon atoms.

## EFFECTS OF THE INVENTION

According to the aspect of the present disclosure, a method for producing an olefin-containing composition, which yields a small amount of paraffins as a by-product, and has an excellent yield of olefins with 2 to 5 carbon atoms, can be provided.

## BRIEF DESCRIPTION OF THE DRAWINGS

[Fig. 1] Fig. 1 is a functional configuration view of an information processing system used in Test Example 1.
[Fig. 2] Fig. 2 is a flowchart of a learning process of a machine learning model using the information processing system of Fig. 1.
[Fig. 3] Fig. 3 is a flowchart of a process of predicting a candidate for a zeolite including a metal cation having desired properties with the information processing system of Fig. 1.

## MODE OF CARRYING OUT THE INVENTION

The present disclosure will be described in detail hereinafter. Embodiments of the present disclosure are not limited by the following description, and can be appropriately modified without departing from the gist of the present disclosure. In the present specification, "to", which indicates a numerical range, encompasses that numerical values described before and after "to" are included as a lower limit and an upper limit.

### (Method for producing olefin-containing composition)

The method for producing the olefin-containing composition according to one embodiment includes a step of decomposing plastic in the presence of a zeolite to produce an olefin-containing composition including an olefin with 2 to 5 carbon atoms (may be referred to as a "decomposition step" hereinafter), and may further include other steps as necessary.

### <Decomposition step>

The decomposition step is a step of decomposing plastic in the presence of a zeolite to produce an olefin-containing composition including an olefin with 2 to 5 carbon atoms.

The zeolite is an MFI zeolite including $Rb^+$. Thus, an olefin-containing composition, which includes a small amount of paraffins as a by-product and has an excellent yield of olefins with 2 to 5 carbon atoms, compared to a known MFI zeolite including no $Rb^+$ in the related art, can be produced.

A usage amount of the zeolite relative to the plastic in the decomposition step is not particularly limited, and may be appropriately selected according to the intended purpose. The usage amount of the zeolite relative to 100 parts by mass of the plastic is preferably 100 parts by mass or less, more preferably 10 parts by mass or greater and 100 parts by mass or less, yet more preferably 13 parts by mass or greater and 28 parts by mass or less, and particularly preferably 15 parts by mass or greater and 25 parts by mass or less. When the usage amount of the zeolite relative to the plastic is 10 parts by mass or greater and 100 parts by mass or less relative to 100 parts by mass of the plastic, the plastic can be suitably decomposed, and a yield of olefins with 2 to 5 carbon atoms and a below-described O/P ratio of the olefin-containing composition are favorable. From the viewpoint of obtaining the effects of the present disclosure with a small usage amount of the zeolite, the upper limit of the usage amount of the zeolite relative to the plastic is particularly preferably 30 parts by mass or less relative to 100 parts by mass of the plastic. Therefore, the usage amount of the zeolite relative to the plastic can also be set to 10 parts by mass or greater and 30 parts by mass or less.

#### <<Olefin-containing composition>>

The olefin-containing composition includes an olefin with 2 to 5 carbon atoms, preferably further includes an aromatic compound, and may further include other components, as necessary.

##### -Olefin with 2 to 5 carbon atoms-

The olefin with 2 to 5 carbon atoms preferably includes, as a main component, an olefin including at least one selected from the group consisting of alkenes and dienes, more preferably at least one selected from the group consisting of an alkene with 2 to 5 carbon atoms and a diene with 3 to 5 carbon atoms, and yet more preferably an alkene with 2 to 5 carbon atoms.

Examples of the olefin with 2 carbon atoms include ethylene.

Examples of the olefin with 3 carbon atoms include propylene.

Examples of the olefin with 4 carbon atoms include 1-butene, cis-2-butene, trans-2-butene, and 2-methylpropene.

Examples of the olefin with 5 carbon atoms include 1-pentene, cis-2-pentene, trans-2-pentene, 2-methyl-1-butene, and 2-methyl-2-butene.

In the present disclosure, the olefin with 2 to 5 carbon atoms may be referred to as a "lower olefin".

The lower olefin serves as a raw material for polyolefin. The polyolefin can be suitably used as a raw material in various fields, such as plastic bags, plastic wrapping films, straws, medical devices, home appliance housings, erasers, hosepipes, tires, tubes, CD cases, food trays, food containers, PET bottles, fibers, and the like.

An amount of the lower olefins in the olefin-containing composition is not particularly limited, and may be appropriately selected according to the intended purpose. The amount of the lower olefins is preferably 30 percent by mass or greater and 100 percent by mass or less, and more preferably 35 percent by mass or greater and 60 percent by mass or less.

-Aromatic compound-

The aromatic compound is not particularly limited, and may be appropriately selected according to the intended purpose. The aromatic compound preferably includes at least one selected from the group consisting of benzene, toluene, ethylbenzene, three positional isomers of xylene (p-xylene, m-xylene, and o-xylene), styrene, and cumene.
In the present disclosure, the aromatic compound may be referred to as "useful aromatics".
The useful aromatics serve as a raw material for resins, and the resins can be suitably used as a raw material in various fields, such as CD cases, food trays, food containers, PET bottles, fibers, and the like.
An amount of the useful aromatics in the olefin-containing composition is not particularly limited, and may be appropriately selected according to the intended purpose. The amount of the useful aromatics is preferably 0 percent by mass or greater and 50 percent by mass or less, more preferably 0.1 percent by mass or greater and 30 percent by mass or less, yet more preferably 1 percent by mass or greater and 20 percent by mass or less, and particularly preferably 1 percent by mass or greater and 10 percent by mass or less.
In the present disclosure, the lower olefins and the useful aromatics may be collectively referred to as "useful components".

--O/P ratio --

In the present disclosure, the fact that an amount of paraffins as a by-product is small and a yield of olefins with 2 to 5 carbon atoms is excellent can be evaluated by determining a ratio of a total yield (%) of an olefin product with 2 to 5 carbon atoms to a total yield (%) of a paraffin product with 2 to 5 carbon atoms, i.e., a ratio [the total yield (%) of the olefin product with 2 to 5 carbon atoms/the total yield (%) of the paraffin product with 2 to 5 carbon atoms](may be referred to as an "O/P ratio" hereinafter). In the present disclosure, the term "paraffin" refers to a saturated aliphatic hydrocarbon with 2 to 5 carbon atoms, preferably a saturated chain aliphatic hydrocarbon with 2 to 5 carbon atoms.
The O/P ratio is not particularly limited, and may be appropriately selected according to the intended purpose, but is preferably 1.0 or greater, and more preferably 1.5 or greater. A higher numerical value of the O/P ratio is more preferable, and therefore the upper limit is not particularly limited. The upper limit is preferably 50 or less, and more preferably 20 or less.
The number of moles of the carbon atoms in each useful component and the O/P ratio can be determined by analyzing a tetrahydrofuran (may be abbreviated as "THF" hereinafter)-soluble product (may be referred to as a "THF-soluble product" hereinafter) from the olefin-containing composition obtained in the decomposition step, specifically, a gaseous product and residues, under the following analysis conditions using a gas chromatography (may be abbreviated as "GC" hereinafter) device equipped with a flame ionization detector, and quantifying each component from a ratio between a peak area of each component and a peak area of an internal standard substance according to an internal standard method. The internal standard substance is not particularly limited, as long as a substance for use is stable under the analysis conditions, and is easily separated from a substance to be analyzed. For example, at least one selected from the group consisting of cyclopentane and t-butyl benzene can be used as the internal standard substance. The GC analysis of the gaseous product and THF-soluble product is performed under the analysis conditions described in Examples.
In the case where the decomposition step is performed in a sealed reaction vessel, the THF-soluble product is obtained by adding THF to the residues in the reaction vessel. In the case where the decomposition step is performed in a continuous or open reaction vessel, the gaseous product and the residues can be recovered not only from the inside of the reaction vessel, but also from the trap outside the reaction vessel, and therefore the THF-soluble product is obtained by adding THF to the residues inside the reaction vessel, and adding THF to the trap and the connecting member between the reaction vessel and the trap.

--Useful component yield--

The useful component yield is preferably 35% or greater, more preferably 40% or greater.
The useful component yield is determined as a ratio of the number of moles of the carbon atoms in the useful components to the number of moles of the carbon atoms in the plastic according to the following equation.

Useful component yield (%) = a mass [g] of the generated useful components calculated by GC analysis/a total mass [g] of a plastic sample $\times$ 100

-Other components-

Examples of the other components in the olefin-containing composition include decomposition products of the plastic other than the zeolite and the useful components, undecomposed plastic, and materials or additives included in the raw materials other than the plastic.

The other components in the olefin-containing composition may be appropriately removed by a method known in the related art when the useful components are recycled.

An amount of the other components in the olefin-containing composition is not particularly limited, as long as the effects of the embodiment of the present disclosure are not adversely affected, and may be appropriately selected according to the intended purpose.

<<MFI zeolite including $Rb^+$>>

The framework structures of the zeolite are stored in a database by International Zeolite Association (may be abbreviated as "IZA" hereinafter), and the IUPAC structure codes (may be abbreviated as "structure codes" hereinafter) of the framework structures are defined. "MFI" is the structure code. The MFI zeolite is preferably ZSM-5 (Zeolite Socony Mobil-5).

The crystal system of the MFI zeolite can be determined by analyzing the MFI zeolite by X-ray diffraction (XRD) under the analysis conditions described in Examples. In addition, the crystal system of the MFI zeolite can also be determined by comparing to XRD patterns disclosed in Collection of simulated XRD powder patterns for zeolites, Fifth revised edition (2007) or XRD patterns disclosed in Zeolite Framework Types on the website of the IZA Structure Commission (http://www.iza-struture.org/databases/).

-Pore size-

The pore size of the MFI zeolite is generally 0.50 nm to 0.60 nm. For example, the pore size of HSZ-800 series, which is commercially available from TOSOH CORPORATION, is 0.58 nm based on a catalog value.

The pore size of the MFI zeolite including $Rb^+$ can be measured according to ISO 15901-3:2007 "Pore size distribution and porosity of solid materials by mercury porosimetry and gas adsorption-Part 3: Analysis of micropores by gas adsorption".

-Molar ratio [$SiO_2/Al_2O_3$]

The molar ratio [$SiO_2/Al_2O_3$] of $SiO_2$ to $Al_2O_3$ of the MFI zeolite including $Rb^+$ is not particularly limited, and may be appropriately selected according to the intended purpose, but is preferably 10 or greater and 10,000 or less, more preferably 20 or greater and 10,000 or less, yet more preferably 20 or greater and 5,000 or less, and particularly preferably 500 or greater and 2,000 or less. When the molar ratio [$SiO_2/Al_2O_3$] is 10 or greater and 10,000 or less, a favorable yield of olefins with 2 to 5 carbon atoms and a favorable O/P ratio of the olefin-containing composition are achieved.

The molar ratio [$SiO_2/Al_2O_3$] can be calculated, for example, by accurately weighing the MFI zeolite including $Rb^+$, completely dissolving the MFI zeolite including $Rb^+$ in an aqueous solution including nitric acid and hydrofluoric acid, measuring a volume of a resultant sample, measuring amounts of Si and Al by ICP optical emission spectrometer (e.g., PlasmaQuant PQ 9000, manufactured by Analytik Jena AG), and calculating a molar ratio from the number of moles of Si and Al calculated from the amounts of Si and Al according to the following equation.

Molar ratio [$SiO_2/Al_2O_3$] = 2 $\times$ (number of moles of Si)/(number of moles of Al)

-BET specific surface area-

The BET specific surface area of the MFI zeolite including $Rb^+$ is not particularly limited, and may be appropriately selected according to the intended purpose, but is preferably 100 $m^2/g$ or greater and 1,000 $m^2/g$ or less, more preferably 100 $m^2/g$ or greater and 250 $m^2/g$ or less, yet more preferably 150 $m^2/g$ or greater and 250 $m^2/g$ or less, and particularly preferably 200 $m^2/g$ or greater and 250 $m^2/g$ or less. When the BET specific surface area is 100 $m^2/g$ or greater and 1,000 $m^2/g$ or less, a yield of olefins with 2 to 5 carbon atoms and the O/P ratio of the olefin-containing composition are favorable. When the BET specific surface area is 100 $m^2/g$ or greater and 250 $m^2/g$ or less, a yield of olefins with 2 to 5 carbon atoms and the O/P ratio of the olefin-containing composition are more favorable.

The BET specific surface area of the MFI zeolite including $Rb^+$ can be measured according to ISO 9277:2010 "Determination of the specific surface area of powder (solids) by gas adsorption".

-Particle size-

In the case where a powdery MFI zeolite containing $Rb^+$ is used as the MFI zeolite including $Rb^+$, a 50% particle size D50 (median diameter) (volume average) of the MFI zeolite containing $Rb^+$ is not particularly limited, and may be appropriately selected according to the intended purpose. From the viewpoint of the BET specific surface area, handling, and the like, the 50% particle size D50 is preferably 1 μm or greater and 500 μm or less, more preferably 2 μm or greater and 350 μm or less, and yet more preferably 2 μm or greater and 100 μm or less.

In the present disclosure, the term "powdery" encompasses at least one selected from the group consisting of primary particles, aggregates of primary particles (secondary particles), granules obtained by granulating primary particles, and granules obtained by granulating secondary particles.

The 50% particle size D50 (volume average) of the MFI zeolite including $Rb^+$ refers to a median diameter measured by a laser diffraction particle size analyzer (e.g., a laser diffraction particle size analyzer SALD-7100, manufactured by Shimadzu Corporation).

The particle shape of the MFI zeolite including $Rb^+$ is not particularly limited. Examples of the particle shape include a spherical shape, an ellipsoidal shape, a crushed shape, a flat shape, an irregular shape, and the like. One of the above shapes may be used, or two or more of the above shapes may be used in combination.

-Method of producing MFI zeolite including $Rb^+$-

The method of producing the MFI zeolite including $Rb^+$ is not particularly limited, and may be appropriately selected from methods known in the related technical field. $Rb^+$ can be introduced by processing an MFI zeolite including no $Rb^+$ with an $Rb^+$ source according to the below-described method of the present disclosure. In another embodiment, for example, a zeolite can be produced by adding $Rb^+$ in advance.

--Method of producing MFI zeolite--

Examples of a typical method of producing an MFI zeolite include a method in which hydrothermal synthesis is performed using a mixture (raw material composition) including a silica source, an alumina source, and optionally an organic structure-directing agent, a hydroxide-containing compound, a fluoride ion-containing compound, water, and the like. After the hydrothermal synthesis, solid-liquid separation, water washing, drying in which moisture is removed, for example, in the air of approximately 50°C to 150°C, and the like may be further performed according to common practice, as necessary. In addition, the organic structure-directing agent may be removed by firing that is performed to the extent that the framework of the zeolite is not collapsed. The firing temperature in this case is preferably 500°C to 600°C.

The silica source is not particularly limited, but is preferably precipitated silica, colloidal silica, fumed silica, silica gel, sodium silicate (e.g., sodium metasilicate, sodium orthosilicate, sodium silicate No. 1, sodium silicate No. 2, sodium silicate No. 3, and sodium silicate No. 4), alkoxysilane (e.g., tetraethoxysilane (TEOS) and trimethylethoxysilane (TMEOS)), or the like, more preferably tetraethoxysilane (TEOS) or trimethylethoxysilane (TMEOS), and yet more preferably tetraethoxysilane (TEOS). The above silica sources may be used alone or in combination.

The alumina source is not particularly limited, but is preferably aluminum chloride, aluminum nitrate, aluminum sulfate, sodium aluminate, aluminum alkoxide (e.g., aluminum isopropoxide or the like), or aluminum hydroxide (e.g., boehmite or the like), and more preferably aluminum nitrate. The above alumina sources may be used alone or in combination.

The organic structure-directing agent is not particularly limited, but a tetrapropyl ammonium compound, such as tetrapropyl ammonium hydroxide or the like, or a tetraethyl ammonium compound, such as tetraethyl ammonium hydroxide or the like, is preferably used. The molar ratio of the organic structure-directing agent to the silica source is preferably 0.01 or greater and 0.60 or less, more preferably 0.03 or greater and 0.40 or less, and yet more preferably 0.05 or greater and 0.35 or less.

The hydroxide-containing compound or the fluoride ion-containing compound is added for the purpose of facilitating crystallization of the zeolite. The hydroxide-containing compound is widely used because the hydroxide-containing compound is inexpensive and easily handled. The hydroxide-containing compound is not particularly limited, as long as the hydroxide-containing compound is alkaline in an aqueous solution.

For example, the organic structure-directing agent can also serve as the hydroxide-containing compound. In this case, a hydroxide of the above-described tetrapropyl ammonium compound or tetraethyl ammonium compound is used. According to the above method, an alkali metal is not introduced into the zeolite, thereby producing a protonic zeolite.

However, an alkali metal ion can be introduced into a zeolite. In the case where it is desired to actively introduce alkali metal ions into a zeolite, in addition to the organic structure-directing agent, a hydroxide of an alkali metal or an alkaline earth metal, preferably a hydroxide of an alkali metal, is added as the hydroxide-containing compound. Examples of the organic structure-directing agent used in this case include tetrapropylammonium bromide and tetraethylammonium bromide. Examples of the hydroxide of the alkali metal include lithium hydroxide, sodium hydroxide, potassium hydroxide, rubidium hydroxide, and cesium hydroxide.

An $Rb^+$ source used when an MFI zeolite is produced by adding $Rb^+$ in advance is not particularly limited, and examples of the $Rb^+$ source include a rubidium compound. Specifically, rubidium hydroxide may be used as the hydroxide, which can also serve as the $Rb^+$ source. As a method of introducing alkali metal ions, such as $Rb^+$, an alkali metal compound other than an oxide may be added. In this case, the hydroxide-containing compound or the fluoride ion-containing compound is additionally added to facilitate crystallization of the zeolite. In the case where alkali metal ions are added using the $Rb^+$ source or another alkali metal compound during production of the zeolite, the alkali metal compound is likely to be excessive, and therefore washing is performed with water after the hydrothermal synthesis, thereby washing off and removing the excessive alkali metal ions. The above $Rb^+$ source is preferably added before the hydrothermal synthesis.

Water used in the hydrothermal synthesis is not particularly limited. Examples of the water include industrial water, tap water, distilled water, deionized water, pure water, RO water (reverse osmosis membrane treated water), and ultrapure water. The above examples of the water may be used alone or in combination.

The hydrothermal synthesis is generally performed in a reaction vessel. As the reaction vessel used in the hydrothermal synthesis, any known reaction vessel known in the related art can be used as long as it is a sealed pressure-resistant vessel used for a hydrothermal synthesis, and a type of the reaction vessel is not particularly limited. For example, a sealed heat-resistant and pressure-resistant vessel, such as an autoclave equipped with a stirring device, a heat source, a pressure gauge, and a safety valve, or the like is preferably used. The crystallization of the zeolite may be performed in a state in which the mixture is left to stand. From the viewpoint of improving uniformity of a resultant zeolite, the crystallization is preferably performed in a state in which the mixture is stirred and mixed.

A processing temperature (reaction temperature) of the hydrothermal synthesis is not particularly limited, and may be appropriately selected from the viewpoint of crystallinity of a resultant zeolite, cost efficiency, and the like. The processing temperature is preferably 100°C or higher and 200°C or lower, more preferably 120°C or higher and 190°C or lower, and yet more preferably 150°C or higher and 180°C or lower.

A processing time (reaction time) of the hydrothermal synthesis is not particularly limited as long as crystallization can be performed, and may be appropriately selected from the viewpoint of crystallinity of a resultant zeolite, cost efficiency, and the like. The processing time is preferably 1 hour or longer and 20 days or shorter, more preferably 4 hours or longer and 15 days or shorter, and yet more preferably 12 hours or longer and 11 days or shorter.

A processing pressure of the hydrothermal synthesis is not particularly limited. The autogenous pressure, which is generated when the mixture in the reaction vessel is heated in the above temperature range, is sufficient to be used as the processing pressure. At this time, an inert gas, such as a nitrogen gas, an argon gas, or the like, may be introduced into the vessel as necessary.

The zeolite may include non-metal cations, such as ammonium ions ($NH_4^+$), protons ($H^+$), and the like. A method of performing ion exchange between the non-metal cations and $Rb^+$ of the $Rb^+$ source is not particularly limited, and can be performed according to common practice.

--Method of producing MFI zeolite including $Rb^+$ using MFI zeolite as raw material--

The method of producing the MFI zeolite including $Rb^+$ of the present disclosure includes a mixing step and a calcination step, preferably further includes a stirring step and an evaporation drying step, and further includes other steps as necessary.

---Mixing step---

The mixing step is a step of obtaining a liquid mixture in which an MFI zeolite (may be referred to as a "raw material zeolite" hereinafter) and a liquid including a rubidium compound are mixed.

By performing the mixing step, for example, a cation ($Rb^+$) included in the rubidium compound can be bonded to an acid site of the raw material zeolite via ionic bonding.

The rubidium compound is not particularly limited. Examples of the rubidium compound include rubidium hydroxide, rubidium nitrate, rubidium chloride, and rubidium carbonate. The above rubidium compounds may be used alone or in combination. Among the above rubidium compounds, the rubidium compound is preferably rubidium nitride because of excellent solubility.

The raw material zeolite used in the mixing step may be an acidic zeolite or a basic zeolite. Between the acidic zeolite and the basic zeolite, an acidic zeolite including Bronsted acid sites is preferably used as the raw material zeolite because ion exchange with cations efficiently progresses when the liquid including the rubidium compound is used.

The raw material zeolite used in the mixing step is not particularly limited, and may be appropriately synthesized or a commercial product.

A method of synthesizing the raw material zeolite is not particularly limited, and may be appropriately selected from methods known in the related art. Examples of the method include a method in which an aluminum nitrate aqueous solution is added to tetraalkoxysilane to carry out hydrolysis, and after removing generated alcohol, a hydrothermal synthesis is performed, followed by performing calcination.

A solvent used in the liquid including the rubidium compound is not particularly limited, but is preferably a solvent that can dissolve the rubidium compound, can be relatively easily removed in a below-described calcination step, and is not decomposed, and more preferably a highly polar solvent. Examples of such a solvent include water, methanol, ethanol, acetonitrile, dimethylformamide, dimethylacetamide, dimethylsulfoxide, and hexamethylphosphoramide. The above solvents may be used alone or in combination. Among the above solvents, at least one selected from the group consisting of water, methanol, and ethanol is preferable in view of a relatively low boiling point and easy removal, and water is particularly preferable in view of low cost.

The concentration of the rubidium compound in the liquid including the rubidium compound is not particularly limited, and may be appropriately selected according to the intended purpose. In view of reduction in cost for the solvent, the concentration is preferably 0.01 mol/L or greater and 10 mol/L or less, more preferably 0.05 mol/L or greater and 2.5 mol/L or less, and more preferably 0.05 mol/L or greater and 0.5 mol/L or less. When the concentration of the rubidium compound is 0.01 mol/L or greater and 10 mol/L or less, a homogeneous liquid mixture including the raw material zeolite and the solution, in which the rubidium compound is dissolved in the solvent, can be obtained, and such a concentration is also advantageous in view of cost.

The amount of substance of the rubidium compound used in the liquid including the rubidium compound can be appropriately determined according to a ratio [(the valence $\times$ the amount of substance) of $Rb^+$ in the rubidium compound in the liquid including the rubidium compound/the quantity of acid sites of the raw material zeolite] of the product of the valence of the cation ($Rb^+$) in the rubidium compound and the amount of substance of the cation ($Rb^+$) in the rubidium compound relative to the quantity of acid sites of the raw material zeolite (i.e., the amount of substance of sites of the raw material zeolite capable of receiving cations ($Rb^+$)). In the case where two or more rubidium compounds are used, the valence of the cation ($Rb^+$) in the rubidium compound is a sum of valences of the cations ($Rb^+$) in the two or more rubidium compounds, and the amount of substance of the cation ($Rb^+$) in the rubidium compound is a sum of the amounts of substance of cations ($Rb^+$) in the two or more rubidium compounds.

The ratio [(the valence $\times$ the amount of substance) of $Rb^+$ in the rubidium compound in the liquid including the rubidium compound/the quantity of acid sites of the raw material zeolite] is not particularly limited, and may be appropriately selected according to the intended purpose. The ratio [(the valence $\times$ the amount of substance) of $Rb^+$ in the rubidium compound in the liquid including the rubidium compound/the quantity of acid sites of the raw material zeolite] is preferably 40 or greater and 4,000 or less, and more preferably 200 or greater and 600 or less. When the ratio [(the valence $\times$ the amount of substance) of $Rb^+$ in the rubidium compound in the liquid including the rubidium compound/the quantity of acid sites of the raw material zeolite] is 40 or greater, cations ($Rb^+$) of the rubidium nitrate can be sufficiently imparted to the sites of the raw material zeolite capable of receiving cations. When the ratio [(the valence $\times$ the amount of substance) of $Rb^+$ in the rubidium compound in the liquid including the rubidium compound/the quantity of acid sites of the raw material zeolite] is 4,000 or less, it is advantageous in view of cost.

The quantity of acid sites in the raw material zeolite can be determined, for example, by a method in which an amount of base adsorbed on the raw material zeolite is measured by temperature programmed deposition (TPD) using an appropriate base (e.g., ammonia).

A method of mixing the raw material zeolite and the liquid including the rubidium compound to obtain the liquid mixture is not particularly limited, and may be appropriately selected according to the intended purpose. Examples of the method include a method in which the raw material zeolite is immersed in a solution in which the rubidium compound is dissolved in a solvent.

The cations included in the raw material zeolite are not particularly limited, but an MFI zeolite including $H^+$ is preferable in view of favorable substitution efficiency with $Rb^+$.

In the case where the MFI zeolite including $H^+$ is used as a raw material of the MFI zeolite including the $Rb^+$, a quantity of acid sites of the MFI zeolite including $H^+$ is not particularly limited, and may be appropriately selected according to the intended purpose. The quantity of acid sites of the MFI zeolite including $H^+$ is preferably 0.01 mmol/g or greater and 2.5 mmol/g or less, more preferably 0.01 mmol/g or greater and 2.0 mmol/g or less, and yet more preferably 0.01 mmol/g or greater and 0.1 mmol/g or less. When the quantity of acid sites of the MFI zeolite including $H^+$ is 0.01 mmol/g or greater and 2.5 mmol/g or less, an MFI zeolite including $Rb^+$, which achieves a favorable yield of olefins with 2 to 5 carbon atoms, and having a favorable O/P ratio of a resultant olefin-containing composition, can be obtained.

The quantity of acid sites of the MFI zeolite including $H^+$ can be measured by ammonia temperature programmed deposition (may be referred to as "TPD" hereinafter).

In the mixing step, a range (range of yz/x (mmol/g)) obtained by dividing the numerical value, which is obtained by multiplying y and z, by x is not limited, where x (g) is a mass of the MFI zeolite, y is a molar ratio [$SiO_2/Al_2O_3$] of $SiO_2$ to $Al_2O_3$ of the MFI zeolite, and z (mmol) is the number of moles of the rubidium compound. The range may be appropriately selected according to the intended purpose. The mixing step preferably satisfies $1 \leq yz/x \leq 10,000$, more preferably $2,000 \leq yz/x \leq 8,000$, and yet more preferably $4,000 < yz/x \leq 6,000$. Since $1 \leq yz/x \leq 10,000$ is satisfied, an effect of improving the O/P ratio of the olefin-containing composition owing to the addition of the MFI zeolite including $Rb^+$ can be sufficiently exhibited, and it is advantageous in view of reduction in labor for preparing a zeolite and raw material cost.

---Stirring step---

The stirring step is a step of stirring the liquid mixture including the raw material zeolite and the liquid including the rubidium compound to obtain the solution-treated zeolite.

A method of stirring the liquid mixture is not particularly limited, and any method known in the related art can be used.

The stirring time of the liquid mixture is not particularly limited, and may be appropriately determined according to the concentration of the rubidium compound in the liquid including the rubidium compound. The stirring time is preferably 0.5 hours to 48 hours, more preferably 1 hour to 24 hours, and yet more preferably 6 hours to 15 hours at room temperature in the air. When the stirring time is 0.5 hours or longer, cations included in the rubidium compound can be sufficiently imparted to the raw material zeolite. When the stirring time is 48 hours or shorter, contamination with substances included in the air, or uneven distribution of cations due to a change in the concentration of the liquid mixture can be prevented.

The solution-treated zeolite, which is obtained by performing the stirring step, is preferably filtered, for example, by a method of performing natural filtration, a method of performing suction filtration, or the like, and washed. Washing of the solution-treated zeolite can be performed, for example, by a method of passing a solvent, which is a same solvent as the solvent used in the solution in which the rubidium compound is dissolved in the solvent, through the solution-treated zeolite collected by the filtration.

---Evaporation drying step---

The evaporation drying step is a step of drying the zeolite by evaporation without filtration of the solution-treated zeolite. Since the evaporation drying step is included, cation exchange can progress further.

A method for the evaporation drying is not particularly limited, and may be appropriately selected according to a type of the solvent included in the liquid mixture. In the case where the solvent is water, examples of the method for the evaporation drying include a method in which the solution-treated zeolite is left to stand in the air within a room temperature environment for an appropriate time. In this case, the time for leaving the solution-treated zeolite to stand is not particularly limited as long as the solvent can be removed, but is preferably 2 days to 14 days, more preferably 3 days to 12 days, and yet more preferably 4 days to 6 days.

The evaporation drying step may be performed by a method in which the solution-treated zeolite is heated in the air for an appropriate time within an oven set at a temperature that is equal to or higher than room temperature and equal to or lower than a boiling point of the solvent, and is a temperature at which bumping of the solvent does not occur. According to this method, the time required to complete evaporation drying can be shortened. The time of heating in the oven can be appropriately adjusted according to the heating temperature. For example, in the case where the solvent is water, the heating may be performed at a temperature of approximately 80°C for 5 hours to 10 hours, or at a temperature of approximately 50°C for 40 hours to 60 hours.

In addition, the solution-treated zeolite recovered by evaporation drying is preferably washed with a solvent that is a same solvent as the solvent used for preparation of the rubidium compound solution, followed by being subjected to a calcination step for removing an excess rubidium compound that is assumed to be deposited on the surface of the solution-treated zeolite.

---Calcination step---

The calcination step is a step of calcining the zeolite obtained after the mixing step, the stirring step, or the evaporation drying step.

In the case where an acidic zeolite is used as the raw material zeolite, as the calcination step is performed, protons at the acid sites of the raw material zeolite and nitric acid ions derived from the rubidium compound, which still remain even after the mixing step and the stirring step, are evaporated as water and nitrogen dioxide, thereby further facilitating incorporation of cations into the zeolite. In addition, the solvent remaining even after the filtration or the evaporation drying step can be almost completely removed.

The conditions and temperature of calcination of the solution-treated zeolite in the calcination step are not particularly limited, but are preferably 300°C to 600°C in the air, and more preferably 450°C to 550°C in the air.

The calcination time of the solution-treated zeolite in the calcination step is not particularly limited, but is preferably 1 hour to 20 hours, and more preferably 6 hours to 15 hours.

The pore size of the MFI zeolite including $Rb^+$ can be controlled by varying the conditions of the mixing step and the stirring step. It is assumed that, as the BET specific area is smaller, a pore size of a resultant zeolite is smaller. Zeolites having different BET specific surface areas can be produced by changing at least one condition selected from the group consisting of: the ratio [(valence × an amount of substance) of $Rb^+$ in the rubidium compound in the liquid including the rubidium compound/a quantity of acid sites of the raw material zeolite] of a product of a valence of the cation in the rubidium compound in the solution and an amount of substance of the cation ($Rb^+$) in the rubidium compound to an amount of substance of sites of the raw material zeolite capable of receiving cations (a quantity of acid sites of the raw material zeolite); the concentration of the rubidium compound in a solution obtained by dissolving the rubidium compound in a solvent; and the stirring time of the liquid mixture.

<<Plastic>>

The plastic is not particularly limited, and may be appropriately selected according to the intended purpose. From the viewpoint of reduction in the environmental loads, the plastic preferably includes plastic waste, more preferably polyolefin, and yet more preferably at least one selected from the group consisting of polyethylene (PE), polypropylene (PP), and polystyrene (PS), which are commonly used in drink and food containers, wrapping materials, molded articles, films, and the like.

In addition to the above-described plastic materials, the plastic may further include other plastic materials, such as polyethylene terephthalate (PET), polybutylene terephthalate (PBT), polyethylene naphthalate (PEN), polycarbonate (PC), polyvinyl chloride, polyvinylidene chloride, chlorinated polyethylene, polyamide, polyurethan, an acrylonitrile-butadiene-styrene copolymer, polymethyl methacrylate, and the like.

In the case where the plastic is plastic waste, a composition ratio of the plastic waste is preferably 20 percent by mass to 40 percent by mass of PE : 20 percent by mass to 40 percent by mass of PP : 10 percent by mass to 30 percent by mass of PS.

An amount of the other plastic materials in the plastic is not particularly limited, and may be appropriately selected according to a type of plastic waste. In view of a yield of olefins with 2 to 5 carbon atoms and an O/P ratio of an olefin-containing composition, the amount of the other plastic materials is preferably 50 percent by mass or less, more preferably 45 percent by mass or less, and yet more preferably 40 percent by mass or less, relative to a total mass of the plastic.

The plastic may be a plastic composition that further includes a material or additive other than the plastic. In the case where the plastic composition is a plastic waste composition, examples of the material other than the plastic include paper and metals.

An amount of the material or additive other than plastic in the plastic composition is not particularly limited.

The decomposition step is preferably performed by heating in view of favorable decomposition efficiency.

A reaction vessel in which the decomposition step is performed is not particularly limited, and may be appropriately selected according to the intended purpose. Examples of the reaction vessel include a batch reactor, a fixed bed reactor, and a fluidized bed reactor.

The temperature at which the decomposition step is performed (may be referred to as a "decomposition temperature" hereinafter) is not particularly limited, and may be appropriately selected according to the intended purpose. The temperature is preferably 300°C or higher and 1,100°C or lower, and more preferably 300°C or higher and 600°C or lower. When the decomposition temperature is 300°C or higher and 1,100°C or lower, the zeolite is easily activated, and a favorable yield of olefins with 2 to 5 carbon atoms and a favorable O/P ratio of an olefin-containing composition can be achieved.

The time for performing decomposition at the decomposition temperature (may be referred to as a "decomposition time" hereinafter) is not particularly limited, and may be appropriately selected according to a reaction system or the like. For example, in the case where the decomposition is performed using a sealed batch reactor, the decomposition time is preferably 1 hour or longer and 20 hours or shorter, and more preferably 5 hours or longer and 10 hours or shorter. When the decomposition time is 1 hour or longer, the plastic can be suitably decomposed, and a favorable yield of olefins with 2 to 5 carbon atoms and a favorable O/P ratio of an olefin-containing composition can be achieved. When the decomposition time is 20 hours or shorter, the decomposition can be performed efficiently.

The atmosphere in which the decomposition is performed is not particularly limited, and may be appropriately selected according to the intended purpose. The decomposition is preferably performed in the presence of an inert gas.

The inert gas is not particularly limited, and may be appropriately selected according to the intended purpose. Examples of the inert gas include a nitrogen gas and an argon gas. The above gases may be used alone or in combination.

In order to completely fluidize the plastic, part of the plastic may be vaporized in the decomposition step. In case of industrial waste, a relatively uniform plastic waste material that is formed of specific plastic components and has a molecular weight distribution within a certain range may be obtained. In such a case, processing conditions of the decomposition step are preferably adjusted according to types of the plastic components constituting the plastic waste material and the molecular weight of the plastic waste material. When inorganic materials are separated from a plastic waste material that is general waste, a separation device of a laboratory scale may be used, but the separation can also be performed at an industrial level in various plants, such as an oil refinery and the like.

<Other steps>

The other steps in the method for producing the olefin-containing composition are not particularly limited, and may be appropriately selected according to the intended purpose. Examples of the other steps include a pretreatment step of the plastic and a recovery step of a volatile component obtained in the decomposition step. In addition, the above-described mixing step, stirring step, and calcination step for producing the MFI zeolite including $Rb^+$ may be included.

<<Pretreatment step>>

The pretreatment step is a step of pretreating the plastic before subjecting the plastic to the decomposition step. Since the plastic is transformed into a form or state that is easily decomposed, the plastic can be decomposed more efficiently.

Examples of the pretreatment include pulverization of the plastic, palletization (chipping) of the pulverized product of the plastic, and melting of the plastic.

The melting of the plastic is preferably performed at lower than 300°C in the absence of the zeolite.

The pulverized product of the plastic is not particularly limited, and may be appropriately selected according to the intended purpose. Examples of the pulverized product include powders and flakes.

The method of obtaining the pulverized product of the plastic is not particularly limited, and may be appropriately selected from methods known in the related art. Examples of the method include a method in which the plastic is pulverized by a pulverizer to obtain a powder or flakes.

The method of pelletizing (chipping) the pulverized product is also not limited, and may be appropriately selected from methods known in the related art. Examples of the method include a method in which, after melting and extruding the pulverized product, the strand-shaped melt-extrusion product is cut to obtain the raw material in the form of chips.

The plastic can be supplied to the decomposition step in the melted state. The method of melting the plastic is not particularly limited, and may be appropriately selected from methods known in the art. Examples of the method include a method in which the plastic is continuously supplied to the decomposition step using a melt-extruder.

<<Volatile component recovery step>>

The volatile component recovery step is a step of recovering the volatile component obtained in the decomposition step.

Inclusion of the volatile component recovery step is advantageous because, when a volatile component is generated in the decomposition step, the volatile component can be collected or removed according to usefulness of the volatile component. For example, in the case where the plastic is polystyrene, styrene monomers generated in the decomposition step can be vaporized and recovered. The recovered styrene monomers are advantageous because the recovered styrene monomers can be reused in production of polystyrene or the like. In the case where the plastic material includes chlorine-containing plastic, such as polyvinyl chloride and polyvinylidene chloride, hydrogen chloride generated when the chlorine-containing plastic is heated in the decomposition step is preferably vaporized and removed. Separately from the decomposition step, the plastic material may be heated at a low temperature in advance to vaporize hydrogen chloride, followed by subjecting the resultant plastic material to the decomposition step. In this case, the temperature for the heating at the low temperature is preferably 100°C or higher and lower than 300°C. Thus, after the plastic is fluidized, the chlorine content of the fluidized olefin-containing composition can be reduced.

The method of recovering the volatile component is not particularly limited, and may be appropriately selected from methods known in the art.

According to the above method for producing the olefin-containing composition, a raw material suitable for chemical recycling, which generates a small amount of paraffins as a by-product and achieves an excellent yield of olefins with 2 to 5 carbon atoms, can be obtained.

(Catalyst for producing olefin-containing composition)

A catalyst for producing an olefin-containing composition according to one embodiment of the present disclosure is a catalyst used for production of an olefin-containing composition including an olefin with 2 to 5 carbon atoms. The catalyst for producing the olefin-containing composition includes an MFI zeolite including $Rb^+$, and may further include other components as necessary.

Examples of a raw material of the olefin-containing composition include plastic.

<MFI zeolite including $Rb^+$>

The MFI zeolite including $Rb^+$ is as described in the above section of (Method for producing olefin-containing composition).

The amount of the MFI zeolite including $Rb^+$ in the catalyst for producing the olefin-containing composition is not particularly limited as long as the effects of the present embodiment are not adversely affected, and may be appropriately selected according to the intended purpose. The catalyst for producing the olefin-containing composition may be the MFI zeolite including $Rb^+$ itself (i.e., the amount of the MFI zeolite including $Rb^+$ being 100 percent by mass).

<Other components>

The other components in the catalyst for producing the olefin-containing composition are not particularly limited. Examples of the other components include a pH adjuster, a preservative or fungicide, a caking additive (binder), and a granulation aid.

The amount of the other components in the catalyst for producing the olefin-containing composition is not particularly limited as long as the effects of the present embodiment are not adversely affected, and may be appropriately selected according to the intended purpose.

The catalyst for producing the olefin-containing composition is a catalyst, specifically, a catalyst including the MFI zeolite including $Rb^+$, which is used for production of an olefin-containing composition including an olefin with 2 to 5 carbon atoms. Accordingly, the method of using the catalyst that includes the MFI zeolite including $Rb^+$ in production of the olefin-containing composition including the olefin with 2 to 5 carbon atoms is also included in the scope of the present disclosure.

Examples

The embodiments of the present disclosure will be concretely described hereinafter through Test Examples, Referential Production Examples, Production Examples, Comparative Production Examples, Examples, and Comparative Examples. However, the embodiments of the present disclosure are not limited by Test Examples, Referential Production Examples, Production Examples, Comparative Production Examples, Examples, and Comparative Examples in any way. In the following description, "%" denotes "percent by mass" unless otherwise specified.

(Test Example 1: Prediction test)

A relationship between a yield of useful components obtained by decomposition of plastic using a zeolite including metal cations as a catalyst and an O/P ratio was predicted using an information processing system. The information processing system was constructed by using parameters of the zeolite, parameters of the metal cation, and parameters of an ion-exchange anion as explanatory variables, and reaction yields of organic compounds as response variables.

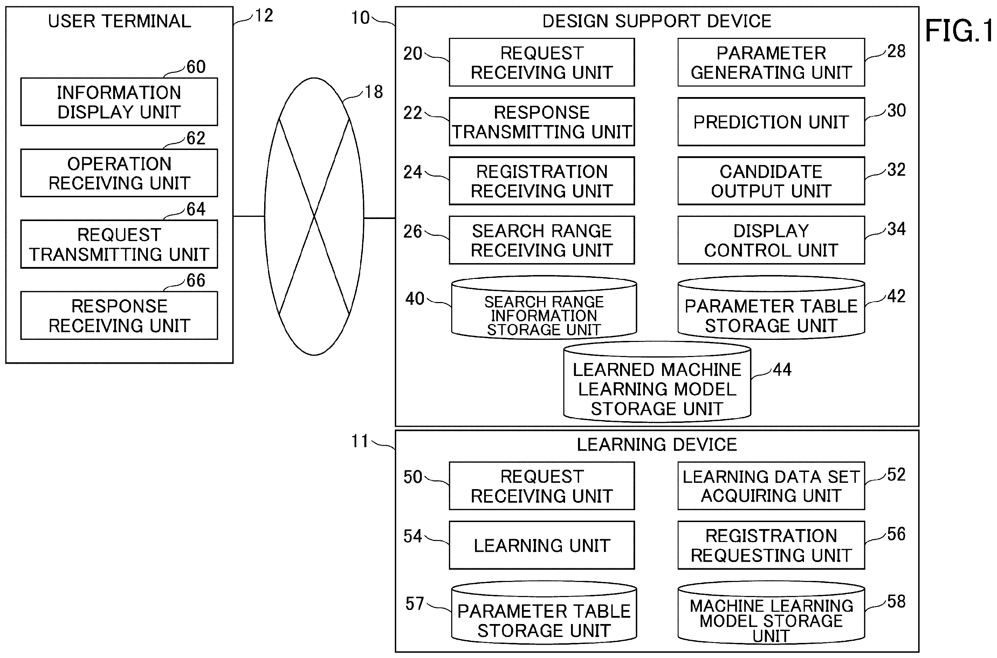

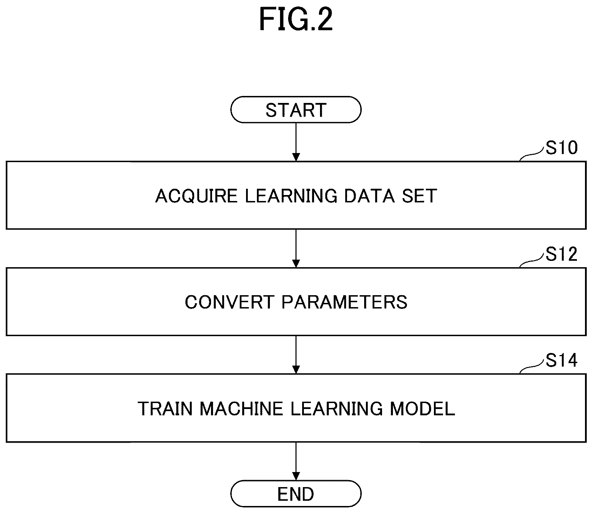

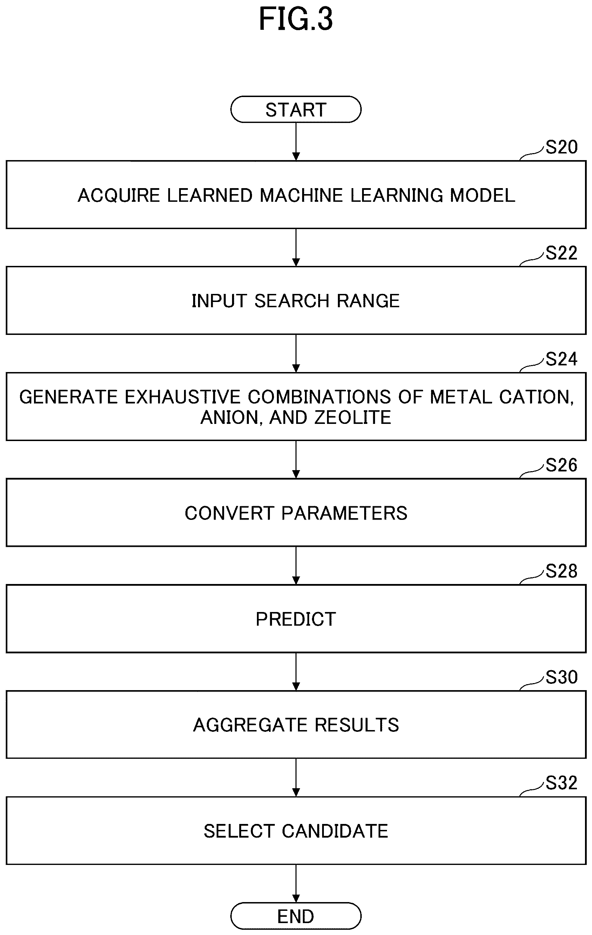

Specifically, a relationship between a yield of useful components obtained by decomposition of plastic when a zeolite including metal cations was used as a catalyst and an O/P ratio was predicted according to the procedure illustrated in Fig. 3 using an information processing system 1 having a functional configuration illustrated in Fig. 1 and using learning processing of a machine learning model in the procedure illustrated in Fig. 2.

The information processing system 1 includes a design support device 10, a learning device 11, and a user terminal 12. The user terminal 12 is an information processing terminal operated by an operator. The design support device 10 is an information processing device, such as PC, for supporting a more efficient design of a zeolite including metal cations, which has desired properties. The design support device 10 generates exhaustive combinations of parameters of the zeolite, parameters of the metal cation, and parameters of an ion-exchange anion, predicts reaction yields of organic compounds corresponding to each of the exhaustive combinations using a learned machine learning model, and outputs a candidate for a metal cation-containing zeolite having desired properties. The learning device 11 is a device that allows the machine learning model to learn a corresponding relationship between information of the parameters of the zeolite, the parameters of the metal cation, and the parameters of the ion-exchange anion and reaction yields of organic compounds.

At step S10 illustrated in Fig. 2, a learning data set acquiring unit 52 of the learning device 11 acquired a learning data set used for learning of the machine learning model. The learning data set was a learning data set representing a corresponding relationship of the parameters of the zeolite, the parameters of the metal cation, and the parameters of the ion-exchange anion relative to reaction yields of organic compounds.

Next, at step S12 illustrated in Fig. 2, the learning data set was converted into a learning data set representing a corresponding relationship of parameters of the zeolite (an item "crystal system"), parameters of the metal cation (items "cation type", "cation ion crystal radius", "cation electronegativity", and "cation charge"), and parameters of the ion-exchange anion (items "ion-exchange anion: $NO_3^-$" and "ion-exchange anion: $OH^-$") relative to reaction yields of organic compounds (items "ethylene yield", "propylene yield", "total yield of C4 olefins and C5 olefins", "useful aromatic yield", and "O/P ratio") using a specific parameter table.

Next, at step S14 illustrated in Fig. 2, a learning unit 54 allowed the machine learning model to learn the corresponding relationship of the parameters of the zeolite, the parameters of the metal cation, and the parameters of the ion-exchange anion relative to the reaction yields of the organic compounds based on the learning data set before the parameter conversion.

Next, the information processing system 1 predicted a candidate for a metal cation-containing zeolite that could give favorable results in the useful component yield and the O/P ratio in the procedure illustrated in Fig. 3.

At step S20, the design support device 10 acquired the learned machine learning model, which had been trained by the learning device 11.

At step S22, a search range receiving unit 26 received an input of a search range of a metal cation-containing zeolite.

At step S24, a parameter generating unit 28 generated exhaustive combinations of parameters of the zeolite, parameters of the metal cation, and parameters of the ion-exchange anion in the search range of the metal cation-containing zeolite input at step S22.

At step S26, the parameter generating unit 28 converted the parameters of the items requiring parameter conversion using a specific parameter table for each of the exhaustive combinations of the parameters of the zeolite, the parameters of the metal cation, and the parameters of the ion-exchange anion generated at step S24. Since a BET specific surface area of a zeolite and a molar ratio [$SiO_2/Al_2O_3$] of the zeolite were not uniquely determined by a zeolite framework, numerical values were separately input.

At step S28, a prediction unit 30 inputs the exhaustive combinations of the parameters of the zeolite, the parameters of the metal cation, and the parameters of the ion-exchange anion generated by the parameter generating unit 28 to the learned machine learning model, thereby predicting reaction yields of organic compounds (items "ethylene yield", "propylene yield", "total yield of C4 olefins and C5 olefins", "useful aromatic yield", and "O/P ratio").

At step S30, a candidate output unit 32 aggregated the reaction yields of the organic compounds corresponding the exhaustive combinations predicted by the prediction unit 30, and sorted the yields in descending order in each item.

At step S32, the candidate output unit 32 outputs the data of a candidate metal cation-containing zeolite based on the reaction yields of the organic compounds corresponding to the exhaustive combinations predicted by the prediction unit 30.

As a result of the prediction test performed by the information processing system 1 above, it was predicted that the zeolite that could provide favorable results in the useful component yield and the O/P ratio was a zeolite having a crystal system of MFI, and cations were $Rb^+$, $Sr^{2+}$, and $Cs^+$.

Based on the prediction results, detailed predictions were further performed on combinations of crystal system of a zeolite, cations, and anions presented in Table 1 below. The results are presented in Table 1 below.

[Table 1]

| Crystal system of zeolite | | MFI | MFI | MFI |
|---|---|---|---|---|
| Cation species | | $Rb^+$ | $Sr^{2+}$ | $Cs^+$ |
| Anion species | | $NO_3^-$ | $NO_3^-$ | $NO_3^-$ |
| Parameters of zeolite | BET specific surface area [$m^2/g$] | 220 | 280 | 220 |
| | Molar ratio [$SiO_2/Al_2O_3$] | 1500 | 1500 | 1500 |
| Parameters of metal cation | Cationic crystal radius [pm] | 166 | 132 | 181 |
| | Cation charge | 1 | 2 | 1 |
| | Cationic element electronegativity | 0.82 | 0.95 | 0.79 |
| Parameters of ion-exchange anion | Parameter | 1 | 1 | 1 |
| Predicted yield [%] | Ethylene | 3 | 4 | 3 |
| | Propylene | 11 | 12 | 11 |
| | C4 olefins and C5 olefins | 10 | 13 | 10 |
| | Useful aromatics | 9 | 10 | 13 |
| | Total of lower olefins | 25 | 28 | 25 |
| | Total of useful components | 33 | 38 | 37 |

(continued)

| Crystal system of zeolite | MFI | MFI | MFI |
|---|---|---|---|
| Predicted O/P ratio | 0.67 | 0.83 | 0.63 |

(Referential Production Example 1)

<Synthesis step of Zeolite a>

In a 200 mL three-necked flask equipped with a stirrer, a condenser, and a dripping funnel, 55 g (0.26 mol) of tetraethoxysilane was weighed. Separately, 0.132 g of aluminum nitrate nonahydrate was dissolved in 10 g of pure water, thereby preparing an aluminum nitrate aqueous solution. While stirring the tetraethoxysilane in the three-necked flask at room temperature (20°C ± 5°C), the entire amount of the aluminum nitrate aqueous solution was added and mixed, thereby preparing a mixture. Subsequently, 85.06 g (0.085 mol) of a 1 mol/L tetrapropylammonium hydroxide aqueous solution was added to the mixture by dripping through a dripping funnel over a course of one hour, while stirring the mixture at room temperature. Thereafter, the reactor was heated in an oil bath set at 100°C and the mixture was stirred for 1 hour, thereby performing hydrolysis of tetraethoxysilane. Subsequently, the condenser of the three-necked flask was replaced with a distillation instrument, the reactor was heated in the oil bath set at 130°C, and ethanol generated by the hydrolysis was removed by distillation for 1.5 hours. During the removal of ethanol by distillation, pure water was added two times at 5 mL per time, and finally a liquid mixture slurry was obtained in the amount of approximately 70 mL. The entire amount of the obtained liquid mixture slurry was placed in an autoclave equipped with an inner cylindrical container formed of Teflon (registered trademark), and a hydrothermal synthesis was performed at 180°C for 11 days. After cooling the resultant slurry to room temperature, the slurry was subjected to centrifugal separation to perform solid-liquid separation. Pure water was added to the obtained solid residue, and the resultant mixture was stirred and then subjected to centrifugal separation to perform washing. This washing was performed twice. The obtained solid residue was dried at 80°C for one day. The obtained dry solid residue was calcined at 550°C for 6 hours in the air to decompose and remove the organic matter, thereby obtaining an MFI zeolite (may be referred to as "Zeolite a" hereinafter).

(Production Example 1)

<Processing step>

Pure water was added to 1.5 g (mass) (10.20 mmol) of rubidium nitrate ($RbNO_3$, purity: 95.0% or higher, manufactured by KANTO CHEMICAL CO., INC.), and the rubidium nitrate was completely dissolved in the pure water, thereby preparing a 0.20 mol/L rubidium nitrate aqueous solution. In a glass container, 5 g of Zeolite a obtained in Referential Production Example 1 and the entire amount of the rubidium nitrate aqueous solution were mixed, and the mixture was stirred at room temperature for 12 hours in the air, thereby obtaining a reaction product.

<Calcination step>

After the processing step, the reaction product was left to stand at room temperature for 5 days in the air to dry the reaction product by evaporation, thereby obtaining a solid product. After washing the obtained solid product with water, the washed solid product was transferred into a porcelain crucible, and was calcined in an electric furnace set at 500°C for 8 hours in the air, thereby obtaining a solid catalyst of Production Example 1.

(Production Example 2)

The processing step and calcination step of the zeolite were performed in the same manner as in Production Example 1 to obtain a solid catalyst of Production Example 2, except that, in the processing step of Production Example 1, the preparation of the 0.20 mol/L rubidium nitrate aqueous solution performed by adding pure water to and completely dissolving 1.5 g (mass) (10.20 mmol) of the rubidium nitrate ($RbNO_3$, purity: 95.0% or higher, manufactured by KANTO CHEMICAL CO., INC.) was changed to preparation of a 0.28 mol/L sodium nitrate aqueous solution performed by adding pure water to and completely dissolving 2.0 g (mass) (13.90 mmol) of rubidium nitrate ($RbNO_3$, purity: 95.0% or higher, manufactured by KANTO CHEMICAL CO., INC.) in the pure water.

(Production Example 3)

The processing step and calcination step of the zeolite were performed in the same manner as in Production Example 1 to obtain a solid catalyst of Production Example 3, except that, in the processing step of Production Example 1, the preparation of the 0.20 mol/L rubidium nitrate aqueous solution performed by adding pure water to and completely dissolving 1.5 g (mass) (10.20 mmol) of the rubidium nitrate ($RbNO_3$, purity: 95.0% or higher, manufactured by KANTO CHEMICAL CO., INC.) was changed to preparation of a 0.37 mol/L sodium nitrate aqueous solution performed by adding pure water to and completely dissolving 2.7 g (mass) (18.60 mmol) of rubidium nitrate ($RbNO_3$, purity: 95.0% or higher, manufactured by KANTO CHEMICAL CO., INC.) in the pure water.

(Comparative Production Example 1)

The synthesis step and the calcination step of the zeolite were performed in the same manner as in Production Example 1 to obtain a solid catalyst of Comparative Production Example 1, except that the processing step was changed as follows.

<Processing step>

Pure water was added to 0.84 g (mass) (9.88 mmol) of sodium nitrate ($NaNO_3$, manufactured by FUJIFILM Wako Pure Chemical Corporation), and the sodium nitrate was completely dissolved in the pure water to prepare a 0.20 mol/L sodium nitrate aqueous solution. In a glass container, 5 g of Zeolite a obtained in Referential Production Example 1 and the entire amount of the sodium nitrate aqueous solution were mixed, and the resultant mixture was stirred at room temperature for 12 hours in the air, thereby obtaining a reaction product.

(Comparative Production Example 2)

The synthesis step and the calcination step of the zeolite were performed in the same manner as in Production Example 1 to obtain a solid catalyst of Comparative Production Example 2, except that the processing step was changed as follows.

<Processing step>

Pure water was added to 1.0 g (mass) (9.89 mmol) of potassium nitrate ($KNO_3$, manufactured by FUJIFILM Wako Pure Chemical Corporation), and the potassium nitrate was completely dissolved in the pure water to prepare a 0.20 mol/L potassium nitrate aqueous solution. In a glass container, 5 g of Zeolite a obtained in Referential Production Example 1 and the entire amount of the potassium nitrate aqueous solution were mixed, and the resultant mixture was stirred at room temperature for 12 hours in the air, thereby obtaining a reaction product.

(Comparative Production Example 3)

The synthesis step and the calcination step of the zeolite were performed in the same manner as in Production Example 1 to obtain a solid catalyst of Comparative Production Example 3, except that the processing step was changed as follows.

<Processing step>

Pure water was added to 1.9 g (mass) (9.75 mmol) of cesium nitrate ($CsNO_3$, manufactured by FUJIFILM Wako Pure Chemical Corporation), and the cesium nitrate was completely dissolved in the pure water to prepare a 0.20 mol/L cesium nitrate aqueous solution. In a glass container, 5 g of Zeolite a obtained in Referential Production Example 1 and the entire amount of the cesium nitrate aqueous solution were mixed, and the resultant mixture was stirred at room temperature for 12 hours in the air, thereby obtaining a reaction product.

(Comparative Production Example 4)

The synthesis step and the calcination step of the zeolite were performed in the same manner as in Production Example 1 to obtain a solid catalyst of Comparative Production Example 4, except that the processing step was changed as follows.

<Processing step>

Pure water was added to 1.0 g (mass) (4.70 mmol) of strontium nitrate ($Sr(NO_3)_2$, manufactured by FUJIFILM Wako Pure Chemical Corporation), and the strontium nitrate was completely dissolved in the pure water to prepare a 0.09 mol/L strontium nitrate aqueous solution. In a glass container, 5 g of Zeolite a obtained in Referential Production Example 1 and the entire amount of the strontium nitrate aqueous solution were mixed, and the resultant mixture was stirred at room temperature for 12 hours in the air, thereby obtaining a reaction product.

(Comparative Production Example 5)

<Synthesis step of titanosilicate>

In a 500 mL three-necked flask equipped with a stirrer, a condenser, and a dripping funnel, 107 g (0.514 mol) of tetraethoxysilane and 4.9 g tetra(n-butoxy) titanium were weighed. Subsequently, 168.1 g (0.168 mol) of a 1 mol/L tetrapropylammonium hydroxide aqueous solution was added to the mixture by dripping over a course of 1.5 hours through the dripping funnel while stirring the mixture over the ice cubes. Thereafter, the reactor was heated in an oil bath set at 100°C, and the mixture was stirred for 1 hour, thereby performing hydrolysis of tetraethoxysilane. Subsequently, the condenser of the three-necked flask was replaced with a distillation instrument, the reactor was heated in the oil bath set at 130°C, and ethanol generated by the hydrolysis was removed by distillation for 1.5 hours. During the removal of ethanol by distillation, pure water was added two times at 5 mL per time, and finally a liquid mixture slurry was obtained in the amount of approximately 70 mL. The entire amount of the obtained liquid mixture slurry was placed in an autoclave equipped with an inner cylindrical container formed of Teflon (registered trademark), and a hydrothermal synthesis was performed at 180°C for 6 days. After cooling the resultant slurry to room temperature, the slurry was subjected to centrifugal separation to perform solid-liquid separation. Pure water was added to the obtained solid residue, and the resultant mixture was stirred and then subjected to centrifugal separation to perform washing. This washing was performed twice. The obtained solid residue was dried at 80°C for one day. The obtained dry solid product was calcined at 550°C for 6 hours in the air to decompose and remove the organic matter, thereby obtaining titanosilicate (may be referred to as "TS-1" hereinafter). TS-1 was determined as a solid catalyst of Comparative Production Example 5.

(Comparative Production Example 6)

The synthesis step and the calcination step of the zeolite were performed in the same manner as in Production Example 1 to obtain a solid catalyst of Comparative Production Example 6, except that the processing step was changed as follows.

<Processing step>

Pure water was added to 0.92 g (mass) (6.24 mmol) of rubidium nitrate ($RbNO_3$, manufactured by KANTO CHEMICAL CO., INC.), and the rubidium nitrate was completely dissolved in the pure water to prepare a 0.12 mol/L rubidium nitrate aqueous solution. In a glass container, 5 g of a faujasite (FAU) zeolite (High silica zeolite HSZ-300, Type 350HUA, manufactured by TOSOH CORPORATION, molar ratio [$SiO_2/Al_2O_3$]: 10) (may be referred to as "Zeolite b" hereinafter) and the entire amount of the rubidium nitrate aqueous solution were mixed, and the resultant mixture was stirred at room temperature for 12 hours in the air, thereby obtaining a reaction product.

(Comparative Production Example 7)

The processing step and calcination steps of the zeolite were performed in the same manner as in Comparative Production Example 1 to obtain a solid catalyst of Comparative Production Example 7, except that, in the processing step of Comparative Production Example 1, the preparation of the 0.2 mol/L sodium nitrate aqueous solution performed by adding pure water to and completely dissolving 0.84 g (mass) (9.88 mmol) of sodium nitrate was changed to preparation of 0.14 mol/L sodium nitrate performed by adding pure water to and completely dissolving 0.61 g (mass) (7.20 mmol) of sodium nitrate, and Zeolite a obtained in Referential Production Example 1 was changed to a commercially-available MFI zeolite (High silica zeolite HSZ-800, Type 840HOA, manufactured by TOSOH CORPORATION, molar ratio [$SiO_2/Al_2O_3$]: 40) (may be referred to as "Zeolite c" hereinafter).

(Comparative Production Example 8)

The processing step and calcination steps of the zeolite were performed in the same manner as in Comparative Production Example 1 to obtain a solid catalyst of Comparative Production Example 8, except that, in the processing step of Comparative Production Example 7, the preparation of the 0.14 mol/L sodium nitrate aqueous solution performed by adding pure water to and completely dissolving 0.61 g (mass) (7.20 mmol) of sodium nitrate in the pure water was changed to preparation of a 0.20 mol/L sodium hydroxide aqueous solution performed by adding pure water to and dissolving 0.40 g (mass) (10.00 mmol) of sodium hydroxide (NaOH, manufactured by FUJIFILM Wako Pure Chemical Corporation) in the pure water.

<Measurement of quantity of acid sites of solid catalyst (zeolite)>

A quantity of acid sites of each of the MFI zeolite (Zeolite a) of Referential Production Example 1, the FAU zeolite (Zeolite b), and the MFI zeolite (Zeolite c) (these are collectively referred to as "raw material zeolites" hereinafter) used as raw materials in the production of the solid catalysts of Production Examples 1 to 3, Comparative Production Examples 1 to 4 and 6 to 8 was analyzed by ammonia temperature programmed deposition (TPD) under the following measuring conditions.

[Measuring conditions of quantity of acid sites]

Device: BEL-CAT-BASIC (manufactured by MicrotracBEL Corp.)
Detector: quadrupole mass analyzer BEL-Mass (manufactured by MicrotracBEL Corp.)
Measurement target species: $NH_3$ (m/z = 16)
Pretreatment: The pretreatment was performed in the order of 1 to 9 in Table 2 below under the conditions presented in Table 1 below.
Heating conditions: The temperature was elevated from 100°C to 600°C at 10°C/min, and kept at 600°C for 30 minutes. The flow rate of the He gas during heating was 30 mL/min.

[Table 2]

| Order of pretreatment | Set temperature [°C] | Set time [min] | Flow gas | Gas flow rate [mL/min] |
|---|---|---|---|---|
| 1 | 25 | 30 | He | 50 |
| 2 | 25~600 | 60 | He | 50 |
| 3 | 600 | 60 | He | 50 |
| 4 | 600~100 | 10 | He | 50 |
| 5 | 100 | 10 | He | 50 |
| 6 | 100 | 30 | 5%$NH_3$/95%He | 50 |
| 7 | 100 | 30 | He | 50 |
| 8 | 100 | 30 | He (Water vapor treatment) | 50 |
| 9 | 100 | 300 | He | 50 |

The quantity of acid sites of the MFI zeolite (Zeolite a) was 0.037 mmol/g, the quantity of acid sites of the FAU zeolite (Zeolite b) was 1.0 mmol/g, and the quantity of acid sites of the MFI zeolite (Zeolite c) was 0.86 mmol/g.

[Evaluation of physical properties of zeolite]

For the zeolites obtained in Referential Production Example 1, Production Examples 1 to 3, and Comparative Production Examples 1 to 8, a crystal system, a BET specific surface area, a molar ratio [$SiO_2/Al_2O_3$], and a salt concentration in an aqueous solution were analyzed by the following methods. In addition, a ratio [(the valence × the amount of substance) of the cation in the salt included in the salt-containing aqueous solution/the quantity of acid sites of the raw material zeolite] of each of the zeolites obtained in Referential Production Example 1, Production Examples 1 to 3, and Comparative Production Examples 1 to 8 was calculated by the following method. The results are presented in Tables 3-1 and 3-2 below.

In Tables 3-1 and 3-2 below, a cationic crystal radius for ions included in each of the zeolites obtained in Referential Production Example 1, Production Examples 1 to 3, and Comparative Production Examples 1 to 4 and 6 to 8 indicates a value of an ionic radius determined by R. D. Shannon and C. T. Prewitt, and the cation element electronegativity indicates a value of Pauling electronegativity.

<Analysis of crystal system of zeolite>

Crystal systems of the zeolites obtained in Referential Production Example 1, Production Examples 1 to 3, and Comparative Production Examples 1 to 8 were analyzed by X-ray diffraction (XRD) under the following measuring conditions.

[X-ray diffraction measuring conditions]

Device: X'Pert Pro MPD (manufactured by Pnalytical)
X-ray source: CuK$\alpha$ rays
Incident X-ray side filter: 10 mm brass mask
Detector-side filter: Ni filter
Incident-side slit: Sollerslit 0.04 rad ASS1/8°
Detector-side slit: ASS 5.0 mm Sollerslit 0.04 rad
Detector: PIXel1D
Measurement method: reflection spectroscopy
Operation range (measuring range): $2\theta$ = 5° to 120°
Step width: 0.006565°
Counting time: 78.795 sec/step

<Measurement of BET specific surface area of zeolite>

BET specific surface areas of the zeolites obtained in Referential Production Example 1, Production Examples 1 to 3, and Comparative Production Example 1 to 8 were measured according to ISO 9277:2010 "Determination of the specific surface area of powder (solids) by gas adsorption".

Specifically, the specific surface area was determined by measuring the zeolite by a specific surface area analyzer (BELSORP (registered trademark) MAX II, manufactured by MicrotracBEL Corp.) at a liquid nitrogen temperature using a nitrogen molecule as a probe according to ISO 9277:2010, and analyzing the result according to the BET method.

<Calculation of molar ratio [$SiO_2/Al_2O_3$] of solid catalyst (zeolite)>

Each of the solid catalysts obtained in Referential Production Example 1, Production Examples 1 to 3, and Comparative Production Examples 1 to 8 was weighed, and the weighed solid catalyst was completely dissolved in an aqueous solution including nitric acid (industrial electronic EL nitric acid 1.38, manufactured by KANTO CHEMICAL CO., INC., purity: 60.0% to 61.0%) and hydrofluoric acid (ultrapure reagent Ultrapur™-100, manufactured by KANTO CHEMICAL CO., INC., purity: 46.0% to 51.0%), and the volume of the resultant solution was fixed, thereby preparing a measurement sample. Amounts of Si and Al in the measurement sample were measured by ICP optical emission spectrometer (PlasmaQuant PQ 9000, manufactured by Analytik Jena AG), and a molar ratio [$SiO_2/Al_2O_3$] was calculated according to the following equation based on the number of moles of Si and Al calculated from the amounts of Si and Al.

Molar ratio [$SiO_2/Al_2O_3$] = 2 $\times$ (number of moles of Si)/(number of moles of Al)

<Measurement of salt concentration of aqueous solution >

The salt concentration of each of the aqueous solutions used in the processing steps of Production Examples 1 to 3 and Comparative Production Examples 1 to 4 and 6 to 8 was determined by molar calculation, assuming that there was no change in the liquid amount due to mixing.

<Calculation of ratio [(valence × the amount of substance) of the cation in the salt included in the salt-containing aqueous solution/the quantity of acid sites in the raw material zeolite]>

The quantity of acid sites in the raw material zeolite used in production of the solid catalysts of Production Examples 1 to 3 and Comparative Production Examples 1 to 4 and 6 to 8 corresponds to an amount of substance of the sites of the raw material zeolite capable of receiving a cation. In addition, a product of a valence and an amount of substance of the cation ($Rb^+$, $Na^+$, $K^+$, $Cs^+$, or $Sr^{2+}$) of the corresponding salt (rubidium nitrate, sodium nitrate, potassium nitrate, cesium nitrate, strontium nitrate, or sodium hydroxide) included in each of the rubidium nitrate aqueous solutions prepared in Production Examples 1 to 3 and Comparative Production Example 6, the sodium nitrate aqueous solutions prepared in Comparative Production Examples 1 and 7, the potassium nitrate aqueous solution prepared in Comparative Production Example 2, the cesium nitrate aqueous solution prepared in Comparative Production Example 3, the strontium nitrate aqueous solution prepared in Comparative Production Example 4, and the sodium hydroxide aqueous solution prepared in Comparative Production Example 8 (these aqueous solutions may be collectively referred to as "salt-containing aqueous solutions" hereinafter) was determined. Subsequently, the ratio [(valence × the amount of substance) of the cation in the salt included in the salt-containing aqueous solution/the quantity of acid sites in the raw material zeolite] of (valence × the amount of substance) of the cation in the salt included in the salt-containing aqueous solution relative to the quantity of acid sites in the raw material zeolite was calculated. The results are presented in Tables 3-1 and 3-2 below.

[Table 3-1]

| | Ref. Pro. Ex. 1 | Pro. Ex. 1 | Pro. Ex. 2 | Pro. Ex. 3 | Comp. Pro. Ex. 1 | Comp. Pro. Ex. 2 |
|---|---|---|---|---|---|---|
| Type of solid catalyst | Zeolite a | Zeolite a | Zeolite a | Zeolite a | Zeolite a | Zeolite a |
| Type of salt | - | $RbNO_3$ | $RbNO_3$ | $RbNO_3$ | $NaNO_3$ | $KNO_3$ |
| Included ion | $H^+$ | $Rb^+$ | $Rb^+$ | $Rb^+$ | $Na^+$ | $K^+$ |
| Crystal system | MFI | MFI | MFI | MFI | MFI | MFI |
| Cationic crystal radius [pm] | -4 | 166 | 166 | 166 | 116 | 152 |
| Cationic element electronegativity | 2.20 | 0.82 | 0.82 | 0.82 | 0.93 | 0.82 |
| Ion-exchange anion | - | $NO_3^-$ | $NO_3^-$ | $NO_3^-$ | $NO_3^-$ | $NO_3^-$ |
| BET specific surface area [$m^2/g$] | 313 | 220 | 122 | 165 | 283 | 298 |
| Mass of raw material zeolite (x) [g] | - | 5 | 5 | 5 | 5 | 5 |
| Molar ratio of raw material zeolite [$SiO_2/Al_2O_3$] (y) | $2.0 \times 10^3$ | $2.0 \times 10^3$ | $2.0 \times 10^3$ | $2.0 \times 10^3$ | $2.0 \times 10^3$ | $2.0 \times 10^3$ |
| Number of moles of salt (z) [mmol] | - | 10.20 | 13.90 | 18.60 | 9.88 | 9.89 |
| Salt concentration of aqueous solution [mol/L] | - | 0.20 | 0.28 | 0.37 | 0.20 | 0.20 |
| yz/x | - | 4080 | 5560 | 7440 | 3952 | 3956 |
| Ratio [(valence × amount of substance) of cation in salt included in salt-containing aqueous solution/ quantity of acid sites of raw material] | - | 276 | 376 | 505 | 267 | 267 |

[Table 3-2]

| | Comp. Pro. Ex. 3 | Comp. Pro. Ex. 4 | Comp. Pro. Ex. 5 | Comp. Pro. Ex. 6 | Comp. Pro. Ex. 7 | Comp. Pro. Ex. 8 |
|---|---|---|---|---|---|---|
| Type of solid catalyst | Zeolite a | Zeolite a | TS-1 | Zeolite b | Zeolite c | Zeolite c |
| Type of salt | $CsNO_3$ | $Sr(NO_3)_2$ | - | $RbNO_3$ | $NaNO_3$ | NaOH |
| Included ion | $Cs^+$ | $Sr^{2+}$ | - | $Rb^+$ | $Na^+$ | $Na^+$ |

(continued)

| | Comp. Pro. Ex. 3 | Comp. Pro. Ex. 4 | Comp. Pro. Ex. 5 | Comp. Pro. Ex. 6 | Comp. Pro. Ex. 7 | Comp. Pro. Ex. 8 |
|---|---|---|---|---|---|---|
| Crystal system | MFI | MFI | MFI | FAU | MFI | MFI |
| Cationic crystal radius [pm] | 181 | 132 | - | 166 | 116 | 116 |
| Cationic element electronegtivity | 0.79 | 0.95 | - | 0.82 | 0.93 | 0.93 |
| Ion-exchange anion | $NO_3^-$ | $NO_3^-$ | - | $NO_3^-$ | $NO_3^-$ | $OH^-$ |
| BET specific surface area [$m^2/g$] | 215 | 289 | 409 | 553 | 241 | 238 |
| Mass of raw material zeolite (x) [g] | 5 | 5 | - | 5 | 5 | 5 |
| Molar ratio of raw material zeolite [$SiO_2/Al_2O_3$] (y) | $2.0 \times 10^3$ | $2.0 \times 10^3$ | - | $1.0 \times 10$ | $4.0 \times 10$ | $2.0 \times 10^3$ |
| Number of moles of salt (z) [mmol] | 9.75 | 4.70 | - | 6.24 | 7.20 | 10.00 |
| Salt concentration of aqueous solution [mol/L] | 0.20 | 0.09 | - | 0.12 | 0.14 | 0.20 |
| yz/x | 3900 | 1880 | - | 12 | 58 | 4000 |
| Ratio [ (valence × amount of substance) of cation in salt included in salt-containing aqueous solution/quantity of acid sites of raw material zeolite] | 263 | 254 | - | 6.24 | 8.37 | 270 |

(Example 1)

Decomposition of a plastic mixture was performed in the presence of the solid catalyst (may be referred to as the "$Rb^+$-substituted MFI zeolite" hereinafter) obtained in Production Example 1 according to the following method.

The mixture including 0.3 g of polyethylene (manufactured by Sigma-Aldrich, the number average molecular weight: 1,700, the weight average molecular weight: 4,000), 0.3 g of polypropylene (manufactured by Sigma-Aldrich, the number average molecular weight: 5,000, the weight average molecular weight: 12,000), 0.3 g of polystyrene (manufactured by Sigma-Aldrich, the weight average molecular weight: 35,000), and 0.2 g of the solid catalyst of Production Example 1 was placed in an inner cylinder formed of quartz. The inner cylinder was sealed in a high-temperature and high-pressure reaction vessel (Model 4790, formed of SUS-316, 100 mL in volume, manufactured by Parr Instrument Company) together with a nitrogen gas at atmospheric pressure, and was heated to an internal temperature of 450°C. The internal temperature was kept for 8 hours. After leaving the resultant product to cool, the gaseous product was recovered, and 10 mL of tetrahydrofuran (THF) (manufactured by KANTO CHEMICAL CO., INC., high-performance liquid chromatography reagent, no stabilizer added) was added to the residue, followed by sufficiently mixing the resultant mixture at room temperature. The solid product was separated by filtration, and the solid residue was washed with a small amount of THF twice. In this manner, a product soluble in THF (THF-soluble product) was extracted in the THF solution obtained by combining the filtrate and the washing liquid.

(Example 2)

A gaseous product and a THF-soluble product were extracted in the same manner as in Example 1, except that the solid catalyst of Production Example 1 was changed to the solid catalyst of Production Example 2 (may be referred to as the "$Na^+$-substituted MFI zeolite" hereinafter).

(Example 3)

A gaseous product and a THF-soluble product were extracted in the same manner as in Example 1, except that the solid catalyst of Production Example 1 was changed to the solid catalyst of Production Example 3 (may be referred to as the "$Na^+$-substituted MFI zeolite" hereinafter).

(Comparative Example 1)

A gaseous product and a THF-soluble product were extracted in the same manner as in Example 1, except that the solid catalyst of Production Example 1 was changed to the solid catalyst of Comparative Production Example 1 (may be referred to as the "$Na^+$-substituted MFI zeolite" hereinafter).

(Comparative Example 2)

A gaseous product and a THF-soluble product were extracted in the same manner as in Example 1, except that the solid catalyst of Production Example 1 was changed to the solid catalyst of Comparative Production Example 2 (may be referred to as the "$K^+$-substituted MFI zeolite" hereinafter).

(Comparative Example 3)

A gaseous product and a THF-soluble product were extracted in the same manner as in Example 1, except that the solid catalyst of Production Example 1 was changed to the solid catalyst of Comparative Production Example 3 (may be referred to as the "$Cs^+$-substituted MFI zeolite" hereinafter).

(Comparative Example 4)

A gaseous product and a THF-soluble product were extracted in the same manner as in Example 1, except that the solid catalyst of Production Example 1 was changed to the solid catalyst of Comparative Production Example 4 (may be referred to as the "$Sr^{2+}$-substituted MFI zeolite" hereinafter).

(Comparative Example 5)

A gaseous product and a THF-soluble product were extracted in the same manner as in Example 1, except that the solid catalyst of Production Example 1 was changed to the solid catalyst (TS-1) of Comparative Production Example 5.

(Comparative Example 6)

A gaseous product and a THF-soluble product were extracted in the same manner as in Example 1, except that the solid catalyst of Production Example 1 was changed to the solid catalyst (may be referred to as the "unsubstituted MFI zeolite" hereinafter) of Referential Production Example 1.

(Comparative Example 7)

A gaseous product and a THF-soluble product were extracted in the same manner as in Example 1, except that the solid catalyst of Production Example 1 was changed to the solid catalyst of Comparative Production Example 6 (may be referred to as the "$Rb^+$-substituted FAU zeolite" hereinafter).

[Evaluation of plastic degradability]

To each of the gaseous products recovered in Examples 1 to 3 and Comparative Examples 1 to 7, 38 mg of cyclopentane (reagent, manufactured by Tokyo Chemical industry Co., Ltd., purity: 98.0% or higher) was added as an internal standard substance, thereby preparing an analysis sample. In addition, to each of the THF-soluble products recovered in Example 1 and Comparative Examples 1 to 7, 0.12 g of t-butylbenzene (reagent, manufactured by Tokyo Chemical industry Co., Ltd., purity: 98.0% or higher) was added as an internal standard substance, thereby preparing an analysis sample.

Each of these analysis samples was analyzed by a gas chromatography (GC) device equipped with a flame ionization detector under the following analysis conditions, to quantify each component from a ratio between the peak area of each component and the peak area of the internal standard substance. The results are presented in Tables 4-1 and 4-2 below.

<<GC analysis conditions of gaseous product>>

Device: Nexis GC-2030 (manufactured by Shimadzu Corporation)
Column: Rt-Alumina BOND (diameter: 0.32 mm, length: 30 m, manufactured by Restek)
Carrier gas type: Ar
Flow rate of carrier gas: 360 mL/min
Injection temperature: 200°C
Sample injection amount: 1 mL
Split ratio: 1/200
Column temperature: Setting a heating program to heat at 120°C (9 minutes), elevate the temperature (10 °C/min), and heat at 200°C (30 minutes)
Detector: flame ionization detector (FID)
Detector temperature: 200°C

<<GC analysis conditions of THF-soluble product>>

Device: Nexis GC-2030 (manufactured by Shimadzu Corporation)
Column: DB-1 (diameter: 0.25 mm, length: 30 m, manufactured by Agilent Technology)
Carrier gas type: He
Flow rate of carrier gas: 97 mL/min
Injection temperature: 350°C
Sample injection amount: 1 μL
Split ratio: 1/50
Column temperature: Setting a heating program to heat at 35°C (10 minutes), elevate the temperature (5 °C/min), and heat at 350°C (10 minutes)
Detector: flame ionization detector (FID)
Detector temperature: 350°C

In Tables 4-1 and 4-2 below, "C4 olefins" encompass 1-butene, cis-2-butene, trans-2-butene, and 2-methylpropane; "C5 olefins" encompass 1-pentene, cis-2-pentene, trans-2-pentene, 2-methyl-1-butene, and 2-methyl-2-butene; "useful aromatics" encompass benzene, toluene, ethylbenzene, three positional isomers (p-xylene, m-xylene, and o-xylene), styrene, and cumene; "lower olefins" encompass olefins with 2 to 5 carbon atoms; "useful components" encompass lower olefins and the useful aromatics; and the "O/P ratio" encompasses a ratio of "the total yield (%) of the olefin product with 2 to 5 carbon atoms/the total yield (%) of the paraffin product with 2 to 5 carbon atoms".

[Table 4-1]

| | | Ex. 1 | Ex. 2 | Ex. 3 | Comp. Ex. 1 | Comp. Ex. 2 |
|---|---|---|---|---|---|---|
| Solid catalyst | | $Rb^+$-substituted MFI zeolite (Production Ex. 1) | $Rb^+$-substituted MFI zeolite (Production Ex. 2) | $Rb^+$-substituted MFI zeolite (Production Ex. 3) | $Na^+$-substituted MFI zeolite (Comp. Production Ex. 1) | $K^+$-substituted MFI zeolite (Comp. Production Ex. 2) |
| Yield [%] | Ethylene | 7 | 5 | 6 | 4 | 3 |
| | Propylene | 21 | 18 | 20 | 13 | 12 |
| | C4 olefins and C5 olefins | 17 | 19 | 18 | 13 | 13 |
| | Useful aromatics | 3 | 4 | 4 | 12 | 11 |
| | Total of lower olefins | 44 | 42 | 44 | 30 | 28 |
| | Total of useful components | 47 | 46 | 47 | 42 | 39 |
| O/P ratio | | 1.57 | 1.52 | 1.62 | 0.92 | 0.80 |

[Table 4-2]

| | | Comp. Ex. 3 | Comp. Ex. 4 | Comp. Ex. 5 | Comp. Ex. 6 | Comp. Ex. 7 |
|---|---|---|---|---|---|---|
| Solid catalyst | | $Cs^+$-substituted MFI zeolite (Comp. Production Ex. 3) | $Sr^{2+}$-substituted MFI zeolite (Comp. Production Ex. 4) | TS-1 (Comp. Production Ex. 5) | Unsubstituted MFI zeolite (Ref. Production Ex. 1) | $Rb^+$-substituted FAU zeolite (Comp. Production Ex. 6) |
| Yield [%] | Ethylene | 3 | 3 | 3 | 4 | 1 |
| | Propylene | 11 | 15 | 10 | 9 | 5 |
| | C4 olefins and C5 olefins | 10 | 17 | 9 | 9 | 9 |
| | Useful aromatics | 7 | 2 | 9 | 16 | 11 |
| | Total of lower olefins | 25 | 35 | 23 | 22 | 15 |
| | Total of useful components | 32 | 36 | 32 | 38 | 25 |
| O/P ratio | | 0.60 | 0.96 | 0.54 | 0.57 | 0.34 |

From the results of Tables 1, 4-1, and 4-2, the MFI zeolite including $Rb^+$ had the most favorable yields of the useful components and the most favorable O/P ratio, among the cations predicted to have favorable results by the prediction model. In addition, the useful component yield and the O/P ratio of the MFI zeolite including $Rb^+$ exceeded the results predicted by the prediction model.

(Test Example 2: Effect of molar ratio [$SiO_2/Al_2O_3$] of solid catalyst)

A gaseous product and a THF-soluble product were extracted in the same manner as in Comparative Example 1, except that the solid catalyst ($Na^+$-substituted MFI zeolite) of Comparative Production Example 1 was changed to the solid catalyst (may be referred to as an "$Na^+$-substituted MFI zeolite" hereinafter) of Comparative Production Example 7.

Each of the components of the obtained gaseous product and the THF-soluble product was quantified from a ratio between the peak area of each component and the peak area of the internal standard substance in the same manner as the evaluation of the plastic degradability in Example 1 and Comparative Examples 1 to 7. The results compared with Comparative Example 1 are presented in Table 5 below.

[Table 5]

| | | Comp. Ex. 1 | Test Ex. 2 |
|---|---|---|---|
| Solid catalyst | | $Na^+$-substituted MFI zeolite (Comp. Production Ex. 1) | $Na^+$-substituted MFI zeolite (Comp. Production Ex. 7) |
| Yield [%] | Ethylene | 4 | 3 |
| | Propylene | 13 | 11 |
| | C4 olefins and C5 olefins | 13 | 14 |
| | Useful aromatics | 12 | 11 |
| | Total of lower olefins | 30 | 27 |
| | Total of useful components | 42 | 38 |
| O/P ratio | | 0.92 | 0.85 |

(Test Example 3: Effect of nitrate aqueous solution in processing step)

A gaseous product and a THF-soluble product were extracted in the same manner as in Comparative Example 1, except that the solid catalyst ($Na^+$-substituted MFI zeolite) of Comparative Production Example 1 was changed to the solid catalyst (may be referred to as a "$Na^+$-substituted MFI zeolite" hereinafter) of Comparative Production Example 8.

Each of the components of the obtained gaseous product and the THF-soluble product was quantified from a ratio between the peak area of each component and the peak area of the internal standard substance in the same manner as the evaluation of the plastic degradability in Example 1 and Comparative Examples 1 to 7. The results compared with Comparative Example 1 are presented in Table 6 below.

[Table 6]

| | | Comp. Ex. 1 | Test Ex. 3 |
|---|---|---|---|
| Solid catalyst | | $Na^+$-substituted MFI zeolite (Comp. Production Ex. 1) | $Na^+$-substituted MFI zeolite (Comp. Production Ex. 8) |
| Yield [%] | Ethylene | 4 | 3 |
| | Propylene | 13 | 8 |
| | C4 olefins and C5 olefins | 13 | 6 |
| | Useful aromatics | 12 | 6 |
| | Total of lower olefins | 30 | 17 |
| | Total of useful components | 42 | 23 |
| O/P ratio | | 0.92 | 0.40 |

(Comparative Example 8)

A gaseous product and a THF-soluble product were extracted in the same manner as in Example 1, except that the solid catalyst of Production Example 1 was changed to the solid catalyst (Zeolite a) of Referential Production Example 1.

(Example 4)

A gaseous product and a THF-soluble product were extracted in the same manner as in Example 1, except that the addition of 0.2 g of the solid catalyst of Production Example 1 to 0.3 g of polyethylene, 0.3 g of polypropylene, and 0.3 g of polystyrene was changed to addition of 0.135 g of the solid catalyst of Production Example 1 to 0.3 g of polyethylene, 3 g of polypropylene, and 0.3 g of polystyrene.

(Example 5)

A gaseous product and a THF-soluble product were extracted in the same manner as in Example 1, except that the addition of 0.2 g of the solid catalyst of Production Example 1 to 0.3 g of polyethylene, 0.3 g of polypropylene, and 0.3 g of polystyrene was changed to addition of 0.225 g of the solid catalyst of Production Example 1 to 0.3 g of polyethylene, 3 g of polypropylene, and 0.3 g of polystyrene.

[Evaluation of plastic degradability]

In the same manner as the evaluation of the plastic degradability in Examples 1 to 3 and Comparative Examples 1 to 7, analysis samples of the gaseous products and the THF-soluble products recovered in Comparative Example 8, Example 4, and Example 5 were prepared, the prepared analysis samples were analyzed by the gas chromatography (GC) device equipped with the flame ionization detector under the above analysis conditions, and each component was quantified from a ratio between the peak area of each component and the peak area of the internal standard substance. The results are presented in Table 7 below. In Table 7, the results of Example 1 are also presented in combination.

In Table 7 below, the "Added amount [phr]" of the solid catalyst denotes parts by mass of the added solid catalyst when the total mass (0.9 g) of polyethylene, polypropylene, and polystyrene is determined as 100 parts by mass.

[Table 7]

| | | Comp. Ex. 8 | Ex. 1 | Ex. 4 | Ex. 5 |
|---|---|---|---|---|---|
| Solid catalyst | Type | Zeolite a (Ref. Production Ex. 1) | $Rb^+$-substituted MFI zeolite (Production Ex. 1) | $Rb^+$-substituted MFI zeolite (Production Ex. 1) | $Rb^+$-substituted MFI zeolite (Production Ex. 1) |
| | Added amount [phr] | 22 | 22 | 15 | 25 |
| Yield [%] | Ethylene | 4 | 7 | 7 | 7 |
| | Propylene | 9 | 21 | 21 | 21 |
| | C4 olefins and C5 olefins | 9 | 17 | 18 | 18 |
| | Useful aromatics | 16 | 3 | 6 | 9 |
| | Total of lower olefins | 22 | 44 | 45 | 45 |
| | Total of useful components | 38 | 47 | 51 | 54 |
| O/P ratio | | 0.57 | 1.57 | 1.14 | 1.34 |

The specific embodiments, production examples, and examples of the present disclosure have been described above, but the present disclosure is not limited by the above embodiments, production examples, and examples. The embodiments may be carried out in various forms, and various combinations, omissions, substitutions, additions, changes, and the like can be made without departing from the gist of the invention. These embodiments and modifications of the embodiments are included in the scope and gist of the invention, and are included in the invention specified in the claims and the equivalent scope of the claims.

The present international application claims priority to Japanese Patent Application No. 2023-131007 filed before the Japan Patent Office on August 10, 2023, the entire contents of which are incorporated herein by reference.

DESCRIPTION OF REFERENCE NUMERALS

1: information processing system
10: design support device
11: learning device
12: user terminal
28: parameter generating unit
30: prediction unit
32: candidate output unit
52: learning data set acquiring unit
54: learning unit

**Claims**

1. A method for producing an olefin-containing composition, the method comprising:
a step of decomposing plastic in the presence of a zeolite to produce an olefin-containing composition including an olefin with 2 to 5 carbon atoms,
wherein the zeolite is an MFI zeolite including $Rb^+$.

2. The method for producing the olefin-containing composition according to claim 1,
wherein the plastic is polyolefin.

3. The method for producing the olefin-containing composition according to claim 1, wherein the plastic includes at least one selected from the group consisting of polyethylene, polypropylene, and polystyrene.

4. The method for producing the olefin-containing composition according to claim 1 or 2, wherein the decomposing of the plastic is performed at a temperature of 300°C or higher and 1,100°C or lower.

5. The method for producing the olefin-containing composition according to claim 1 or 2, wherein a molar ratio [$SiO_2/Al_2O_3$] of $SiO_2$ to $Al_2O_3$ of the MFI zeolite including $Rb^+$ is 10 or greater and 10,000 or less.

6. The method for producing the olefin-containing composition according to claim 1 or 2, wherein the MFI zeolite including $Rb^+$ has a BET specific surface area of 100 $m^2/g$ or greater and 1,000 $m^2/g$ or less.

7. The method for producing the olefin-containing composition according to claim 1, further comprising:

a mixing step of obtaining a liquid mixture in which an MFI zeolite and a liquid including a rubidium compound are mixed; and
a calcination step of calcining the liquid mixture in the air to obtain the MFI zeolite including $Rb^+$.

8. The method for producing the olefin-containing composition according to claim 7, wherein the mixing step satisfies $1 \leq yz/x \leq 10{,}000$, where x (g) is a mass of the MFI zeolite, y is a molar ratio [$SiO_2/Al_2O_3$] of $SiO_2$ to $Al_2O_3$ of the MFI zeolite, and z (mmol) is a number of moles of the rubidium compound.

9. The method for producing the olefin-containing composition according to claim 7, wherein the MFI zeolite is an MFI zeolite including $H^+$.

10. A catalyst for producing an olefin-containing composition, the catalyst comprising:
an MFI zeolite including $Rb^+$,
wherein the catalyst is used for producing an olefin-containing composition that includes an olefin with 2 to 5 carbon atoms.

FIG.1
USER TERMINAL
12
60
INFORMATION DISPLAY UNIT
62
OPERATION RECEIVING UNIT
64
REQUEST TRANSMITTING UNIT
66
RESPONSE RECEIVING UNIT
18
10
DESIGN SUPPORT DEVICE
20
REQUEST RECEIVING UNIT
28
PARAMETER GENERATING UNIT
22
RESPONSE TRANSMITTING UNIT
30
PREDICTION UNIT
24
REGISTRATION RECEIVING UNIT
32
CANDIDATE OUTPUT UNIT
26
SEARCH RANGE RECEIVING UNIT
34
DISPLAY CONTROL UNIT
40
SEARCH RANGE INFORMATION STORAGE UNIT
42
PARAMETER TABLE STORAGE UNIT
44
LEARNED MACHINE LEARNING MODEL STORAGE UNIT
11
LEARNING DEVICE
50
REQUEST RECEIVING UNIT
52
LEARNING DATA SET ACQUIRING UNIT
54
LEARNING UNIT
56
REGISTRATION REQUESTING UNIT
57
PARAMETER TABLE STORAGE UNIT
58
MACHINE LEARNING MODEL STORAGE UNIT

FIG.2

START
S10
ACQUIRE LEARNING DATA SET
S12
CONVERT PARAMETERS
S14
TRAIN MACHINE LEARNING MODEL
END

# FIG.3

START
S20
ACQUIRE LEARNED MACHINE LEARNING MODEL
S22
INPUT SEARCH RANGE
S24
GENERATE EXHAUSTIVE COMBINATIONS OF METAL CATION, ANION, AND ZEOLITE
S26
CONVERT PARAMETERS
S28
PREDICT
S30
AGGREGATE RESULTS
S32
SELECT CANDIDATE
END

## INTERNATIONAL SEARCH REPORT

International application No. **PCT/JP2024/028573**

**A. CLASSIFICATION OF SUBJECT MATTER**

***C10G 1/10***(2006.01)i; ***B01J 29/40***(2006.01)i; ***B01J 37/04***(2006.01)i; ***B01J 37/08***(2006.01)i; ***C08J 11/16***(2006.01)i
FI: C10G1/10; B01J29/40 M ZAB; B01J37/04 102; B01J37/08; C08J11/16

According to International Patent Classification (IPC) or to both national classification and IPC

**B. FIELDS SEARCHED**

Minimum documentation searched (classification system followed by classification symbols)

C10G1/10; B01J29/40; B01J37/04; B01J37/08; C08J11/16

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 6-220458 A (HITACHI, LTD.) 09 August 1994 (1994-08-09) | 1-10 |
| A | JP 2019-527758 A (SABIC GLOBAL TECHNOLOGIES B.V.) 03 October 2019 (2019-10-03) | 1-10 |
| A | JP 2023-532532 A (CHINA PETROLEUM & CHEMICAL CORPORATION) 28 July 2023 (2023-07-28) | 1-10 |
| A | WO 2023/064741 A1 (BOARD OF SUPERVISORS OF LOUISIANA STATE UNIVERSITY AND AGRICULTURAL AND MECHANICAL COLLEGE) 20 April 2023 (2023-04-20) | 1-10 |
| P, X | JP 2024-80690 A (SK INNOVATION CO., LTD.) 13 June 2024 (2024-06-13) claims, paragraph [0046] | 1-4, 9 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 October 2024** | **15 October 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/028573**

| Patent document cited in search report | Publication date (day/month/year) | Patent family member(s) | Publication date (day/month/year) |
|---|---|---|---|
| JP 6-220458 A | 09 August 1994 | (Family: none) | |
| JP 2019-527758 A | 03 October 2019 | US 2019/0299491 A1<br>WO 2018/025104 A1<br>EP 3491103 A1<br>CN 109563413 A | |
| JP 2023-532532 A | 28 July 2023 | US 2023/0357644 A1<br>WO 2022/002091 A1<br>EP 4174122 A1<br>CN 113862018 A<br>TW 202202607 A | |
| WO 2023/064741 A1 | 20 April 2023 | (Family: none) | |
| JP 2024-80690 A | 13 June 2024 | US 2024/0182384 A1<br>claims, paragraph [0047]<br>EP 4379020 A1<br>KR 10-2024-0083186 A<br>CN 118126746 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021166854 A
- JP 2023131007 A


### Non-patent literature cited in the description


- **YOSHIO UEMICHI** ; **YASUHARU KANDA**. *Hydrogen Transfer Degradation of Mixed Plastics Using Zeolite Catalysts*, 2023, vol. 65 (2), 114-119